# EUROPEAN PATENT SPECIFICATION

(11) **EP 3 962 366 B1**
(45) Date of publication and mention of the grant of the patent: **09.12.2026**
(21) Application number: 20819595.8
(22) Date of filing: 04.06.2020
(51) Int. Cl.: A61B 10/00, A61B 10/02, A61B 17/00, A61B 17/34, A61B 17/32, A61B 90/00

(54) **MEDICAL INSTRUMENT FOR INTERVENTIONAL RADIOLOGY PROCEDURE**
MEDIZINISCHES INSTRUMENT FÜR INTERVENTIONELLES RADIOLOGISCHES VERFAHREN
INSTRUMENT MÉDICAL POUR PROCÉDURES DE RADIOLOGIE INTERVENTIONNELLE

(30) Priority: 04.06.2019 US 201962857063 P
(43) Date of publication of application: 09.03.2022
(73) Proprietor: Great Plains Imaging LLC, Wichita, KS 67208 (US)
(72) Inventor: MCFARLAND, Robert, Wichita, Kansas 67208 (US); WHITE, Joel, Wichita, Kansas 67218 (US)
(74) Representative: Loo, Chi Ching
(86) International application number: PCT/US2020/036159
(87) International publication number: WO 2020/247656

(56) References cited:
- EP-A1- 0 704 189
- WO-A1-2016/176403
- WO-A1-2016/201383
- US-A1- 2006 173 377
- US-A1- 2013 310 750
- US-A1- 2015 342 756
- US-B1- 6 572 593
- US-B2- 10 231 716
- US-B2- 7 004 927
- US-B2- 8 808 198
- US-B2- 9 072 823

## Description

### CROSS-REFERENCE TO RELATED APPLICATION

This application claims priority to U.S. Provisional Patent Application No. 62/857,063, filed June 4, 2019, and entitled MEDICAL INSTRUMENT FOR INTERVENTIONAL RADIOLOGY PROCEDURE.

### FIELD

The present disclosure generally relates to medical instruments and procedures associated with interventional radiology, and more specifically, to medical instruments designed to enable radiologists to perform interventional radiology procedures on soft tissue.

### BACKGROUND

Interventional radiology is a radiology specialty in which minimally invasive procedures are performed using image guidance. Interventional radiology procedures may be performed for a variety of reasons. For example, some interventional radiology procedures are done for diagnostic purposes (e.g., biopsy). Other interventional radiology procedures are performed for treatment purposes (e.g., radiofrequency ablation).

During an interventional radiology procedure, a radiologist uses images as guidance for operating with medical instruments. Common interventional imaging methods include, for example, X-ray fluoroscopy, computed tomography (CT), ultrasound, and magnetic resonance imaging (MRI). Medical instruments used in interventional radiology procedures typically include, for example, needles, catheters, drains, and guide-wires. The medical instruments are inserted into a patient's body through the skin, through a body cavity, or through an anatomical opening. The use of an imaging method allows the radiologist to guide these medical instruments through the body to a specific area of interest.

Medical instruments specifically designed for performing interventional radiology procedures are needed. In some instances, these specifically designed medical instruments will enable radiologists to perform new interventional radiology procedures. In other instances, the specifically designed medical instruments will enable radiologists to perform current interventional radiology procedures in a more advanced and/or more efficient manner. Additionally, imaging methods associated with interventional radiology procedures continue to advance because of technological improvements in underlying imaging equipment. Medical instruments specifically designed for performing interventional radiology procedures will also enable radiologists to capitalize on these imaging advancements.

Interventional radiology enables a radiologist to precisely focus on a specific area of interest of the human anatomy. One area of the human anatomy relevant to interventional radiology techniques are hands, wrists, feet, and ankles. Some common conditions/syndromes associated with this area of the human anatomy include, for example, carpal tunnel syndrome, De Quervain's syndrome, trigger fingers, Dupuytren contracture, fibromas, tarsal tunnel syndrome, and cuboid syndrome. The ability to address these conditions/syndromes using interventional radiology procedures will likely enable patients to avoid having to undergo open surgery, which has numerous benefits. For example, a radiologist using an interventional radiology procedure has the ability to visualize internal anatomy of a patient without a large incision, which makes interventional radiology less invasive and less prone to risk of infection than an open surgery. Thus, interventional radiology enables procedures to be performed outside of a traditional hospital setting, significantly reducing the cost of diagnosis or treatment. Additionally, interventional radiology procedures have the potential to decrease the recovery time for a patient because of the less-invasive nature of the procedure.

Accordingly, there is a need for specifically designed medical instruments for performing interventional radiology procedures. There is also a need for new interventional radiology procedures to be developed that take advantage of the specifically designed medical instruments. In particular, there is a need for specifically designed medical instruments for performing interventional radiology procedures that focus on hands, wrists, feet, and ankles. WO 2016/176403 is considered the closest prior art to the invention and discloses a stylet formed with an extension having a shape adapted to collect a diagnostic sample of a target tissue and a method of using the same.

### SUMMARY

In one aspect, a medical instrument for cutting soft tissue during an interventional radiology procedure comprises a hypodermic needle having an inner needle surface, an outer needle surface, a proximal needle end, a distal needle end, and a needle axis. The inner needle surface defines a needle bore extending from the proximal needle end to the distal needle end. The distal needle end has a sharpened distal tip configured to puncture soft tissue. A stylet has a stylet body, a stylet head, an outer stylet surface, a proximal stylet end, a distal stylet end, and a stylet axis. The stylet axis is coaxial with the needle axis. The stylet head is located at the distal stylet end. At least a portion of the stylet is located within the needle bore. The portion of the stylet located within the needle bore and the inner needle surface of the hypodermic needle collectively form at least one fluid passageway. The stylet is movable relative to the hypodermic needle along the needle axis. The stylet is adjustable between a retracted configuration and a protracted configuration. The stylet head is located within the needle bore when the medical instrument is in the retracted configuration. The stylet head is located external to the needle bore when the medical instrument is in the protracted configuration. The fluid passageway enables fluid to flow from the proximal needle end to the distal needle end when the stylet is in the retracted configuration and in the protracted configuration. The fluid passageway is configured such that fluid in the fluid passageway flows between the outer stylet surface and the inner needle surface; wherein the sharpened distal tip enables the hypodermic needle to easily puncture the skin of a patient to gain access to the human anatomy; and the medical instrument is configured such that unlike open surgery, including less invasive surgical procedures that use smaller incisions on the order of 4 mm or greater, an entry point used to conduct the procedure with the medical instrument is only a hypodermic needle puncture having a maximum transverse dimension of less than 3.5 mm.

A medical instrument in accordance with one or more aspects of the present disclosure can be used to conduct numerous interventional radiology procedures including, for example, a carpal tunnel release, a De Quervain release, a trigger finger release, a tarsal tunnel release, a plantar fascia release, a fasciotomy, a lavage (e.g., a shoulder lavage), and a tissue biopsy.

Other aspects will be in part apparent and in part pointed out hereinafter.

### BRIEF DESCRIPTION OF THE DRAWINGS

Figure 1 is a perspective view of a medical instrument in accordance with the present disclosure, the medical instrument being in a retracted configuration.
Figure 2 is a perspective view of the medical instrument shown in Figure 1, the medical instrument being in a protracted configuration.
Figure 3 is a side view of a hypodermic needle in accordance with the present disclosure.
Figure 4 is a cross-sectional view of the hypodermic needle taken along line 4-4 in Figure 3.
Figure 5 is a side view of a stylet in accordance with the present disclosure.
Figure 6 is a cross-sectional view of the stylet taken along line 6-6 in Figure 5.
Figure 7 is an alternative cross-sectional view of the stylet in accordance with the present disclosure.
Figure 8 is a cross-sectional view of the medical instrument taken along line 8-8 in Figure 1.
Figure 9 is a perspective view of a hypodermic needle subassembly in accordance with the present disclosure.
Figure 10 is a side-view of a collar of the hypodermic needle subassembly in accordance with the present disclosure.
Figure 11 is a cross-sectional view of the collar shown in Figure 10, the cross-section being taken along a vertical plane oriented halfway through the collar.
Figure 12 is a perspective view of a stylet subassembly in accordance with the present disclosure.
Figure 13 is a side-view of a fluid fitting of the stylet subassembly in accordance with present disclosure.
Figure 14 is a cross-sectional view of the fluid fitting shown in Figure 13, the cross-section being taken along a vertical plane oriented halfway through the fluid fitting.
Figure 15 is a cross-sectional view illustrating how the fluid fitting is oriented within the collar when the medical instrument is in the retracted configuration, the cross-section being taken along a vertical plane oriented halfway through the fluid fitting and the collar.
Figure 16a is a cross-sectional view illustrating how the fluid fitting is oriented within the collar when the medical instrument is in the protracted configuration, the cross-section being taken along a vertical plane oriented halfway through the fluid fitting and the collar.
Figure 16b is a perspective cross-sectional view of the medical instrument illustrating how the fluid fitting, the collar and the removable clip interact with each other when the medical instrument is in the retracted configuration, the cross-section being taken along a vertical plane oriented halfway through the fluid fitting and the collar.
Figure 17 is a perspective cross-sectional view of the medical instrument illustrating how the fluid fitting, the collar and the removable clip interact with each other when the medical instrument is in the protracted configuration, the cross-section being taken along a vertical plane oriented halfway through the fluid fitting and the collar.
Figure 18 is a cross-sectional view of the fluid fitting taken along the line 18-18 in Figure 13.
Figure 19 is a cross-sectional view of the medical instrument with the stylet removed, the cross-section illustrating the flow of fluid from a syringe and through the hypodermic needle.
Figure 20 is a side view of a locking mechanism, the fluid fitting, and the collar, the figure showing the orientation of the various components when the medical instrument is in the retracted configuration and the locking mechanism is engaged.
Figure 21 is a side view of the locking mechanism, the fluid fitting, and the collar, the figure showing the orientation of the various components when the medical instrument is in the protracted configuration and the locking mechanism is disengaged.
Figure 22a is a perspective view of a first embodiment of a stylet head in accordance with the present disclosure.
Figure 22b is a right side view of the first embodiment of the stylet head shown in Figure 22a, the left side view being a mirror image thereof
Figure 22c is a second perspective view of the first embodiment of the stylet head shown in Figures 22a-22b.
Figure 23a is a perspective view of a second embodiment of a stylet head in accordance with the present disclosure.
Figure 23b is a second perspective view of the second embodiment of the stylet head shown in Figure 23a.
Figure 23c is a right side view of the second embodiment of the stylet head shown in Figures 23a-23b, the left side view being a mirror image thereof.
Figure 23d is a top view of the second embodiment of the stylet head shown in Figures 23a-23c.
Figure 24a is top view of a third embodiment of a stylet head in accordance with the present disclosure.
Figure 24b is a perspective view of the third embodiment of the stylet head shown in Figure 24a.
Figure 24c is a right side view of the third embodiment of the stylet head shown in Figures 24a-24b, the left side view being a mirror image thereof.
Figure 25a is a perspective view of a fourth embodiment of a stylet head in accordance with the present disclosure.
Figure 25b is a right side view of the fourth embodiment of the stylet head shown in Figure 25a, the left side view being a mirror image thereof.
Figure 25c is a second perspective view of the fourth embodiment of the stylet head shown in Figures 25a-b.
Figure 26a is a right side view of a fifth embodiment of a stylet head in accordance with the present disclosure, the left side view being a mirror image thereof.
Figure 26b is a perspective view of the fifth embodiment of the stylet head shown in Figure 26a.
Figure 26c is a second perspective view of the fifth embodiment of the stylet head shown in Figures 26a-26b.
Figure 27a is a right side view of a sixth embodiment of a stylet head in accordance with the present disclosure, the left side view being a mirror image thereof.
Figure 27b is a perspective view of the sixth embodiment of the stylet head shown in Figure 27a.
Figure 27c is a second perspective view of the sixth embodiment of the stylet head shown in Figures 27a-27b.
Figure 28a is a perspective view of a seventh embodiment of a stylet head in accordance with the present disclosure.
Figure 28b is a second perspective view of the seventh embodiment of the stylet head shown in Figure 28a.
Figure 28c is a right side view of the seventh embodiment of the stylet head shown in Figures 28a-28b, the left side view being a mirror image thereof
Figure 29a is a right side view of an eighth embodiment of a stylet head in accordance with the present disclosure, the left side view being a mirror image thereof.
Figure 29b is a perspective view of the eighth embodiment of the stylet head shown in Figure 29a.
Figure 29c is a perspective view of an alternative embodiment of the eight embodiment of the stylet head.
Figure 30a is a perspective view of a ninth embodiment of a stylet head in accordance with the present disclosure.
Figure 30b is a top view of the ninth embodiment of the stylet head shown in Figure 30a, the bottom view being a mirror image thereof.
Figure 30c is a right side view of the ninth embodiment of the stylet head shown in Figures 30a-30b, the left side view being a mirror image thereof.
Figure 31a is a perspective view of a tenth embodiment of a stylet head in accordance in accordance with the present disclosure.
Figure 31b is a right side view of the tenth embodiment of the stylet head shown in Figure 31a.
Figure 31c is a left side view of the tenth embodiment of the stylet head shown in Figures 31a-31b.
Figure 31d is a top view of the tenth embodiment of the stylet head shown in Figures 31a-31c, the bottom view being a mirror image thereof.
Figure 32a is a perspective view of an eleventh embodiment of a stylet head in accordance with the present disclosure.
Figure 32b is a top view of the eleventh embodiment of the stylet head shown in Figure 32a.
Figure 32c is a right side view of the eleventh embodiment of the stylet head shown in Figures 32a-32b, the left side view being a mirror image thereof
Figure 32d is a second perspective view of the eleventh embodiment of the stylet head shown in Figures 32a-32c.
Figure 33 is an image of a patient's wrist showing anatomical structures relevant to carpal tunnel syndrome.
Figure 34 is a side view illustration of an affected wrist in a palmar position.
Figure 35 is a side view illustration of the affected wrist shown in Figure 34 with an ultrasound transducer positioned on the wrist.
Figure 36 is a top view illustration of the affected wrist shown in Figure 34, the affected writing being oriented in the palmar position.
Figure 37 is an illustration showing a hypodermic needle being inserted into a patient's hand/wrist bevel-up in a proximal-to-distal direction relative to the patient's hand/wrist, wherein the needle is enlarged relative the illustrated hand/wrist for ease of understanding.
Figure 38 is an illustration showing a hypodermic needle being inserted into a patient's hand/wrist bevel-up in a distal-to-proximal direction relative to the patient's hand/wrist, wherein the needle is enlarged relative the illustrated hand/wrist for ease of understanding.
Figure 39 is an illustration showing a hypodermic needle being inserted into a patient's hand/wrist bevel-down in a proximal-to-distal direction relative to the patient's hand/wrist, wherein the needle is enlarged relative the illustrated hand/wrist for ease of understanding.
Figure 40 is an illustration showing a hypodermic needle being inserted into a patient's hand/wrist bevel-down in a distal-to-proximal direction relative to the patient's hand/wrist, wherein the needle is enlarged relative the illustrated hand/wrist for ease of understanding.
Figure 41 is an ultrasound image of a carpal tunnel after a fluid pocket is formed between a TCL and a median nerve.
Figure 42 is an ultrasound image showing anatomical structures relevant to carpal tunnel syndrome.

Reference is made in the following detailed description of preferred embodiments to accompanying drawings, which form a part hereof, wherein like numerals may designate like parts throughout that are corresponding and/or analogous. It will be appreciated that the figures have not necessarily been drawn to scale, such as for simplicity and/or clarity of illustration. For example, dimensions of some aspects may be exaggerated relative to others. Further, it is to be understood that other embodiments may be utilized. Furthermore, structural and/or other changes may be made without departing from claimed subject matter. References throughout this specification to "claimed subject matter" refer to subject matter intended to be covered by one or more claims, or any portion thereof, and are not necessarily intended to refer to a complete claim set, to a particular combination of claim sets (e.g., method claims, apparatus claims, etc.), or to a particular claim.

### DETAILED DESCRIPTION

### Overview of Medical Instrument

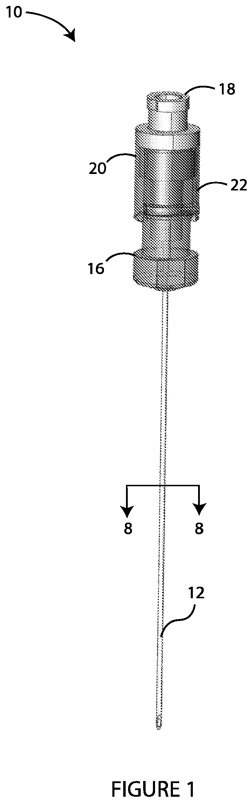

A medical instrument for use during an interventional radiology procedure in accordance with the present disclosure is generally indicated by reference number 10 in Figures 1-2. The medical instrument 10 can be used for manipulating (e.g. moving or cutting) soft tissue during an interventional radiology procedure. The medical instrument 10 includes a hypodermic needle 12 and a stylet 14. A person of ordinary skill in the art will understand that the hypodermic needle 12 and the stylet 14 could be fabricated with several types of materials suitable for medical use within a human patient (i.e., biocompatible). For example, the hypodermic needle 12 could be manufactured from a metal material (e.g., stainless steel, titanium, Nitinol, or tungsten carbide) or a ceramic material (e.g., zirconia, alumina, or sapphire). Similarly, the stylet 14 could be manufactured from a metal material (e.g., stainless steel, titanium, Nitinol, or tungsten carbide) or a ceramic material (e.g., zirconia, alumina, or sapphire). A person of ordinary skill in the art will understand that the material used for fabricating the hypodermic needle 12 and the stylet 14 will depend upon a number of variables, including the type of interventional radiology procedure being performed and the purpose of the interventional radiology procedure.

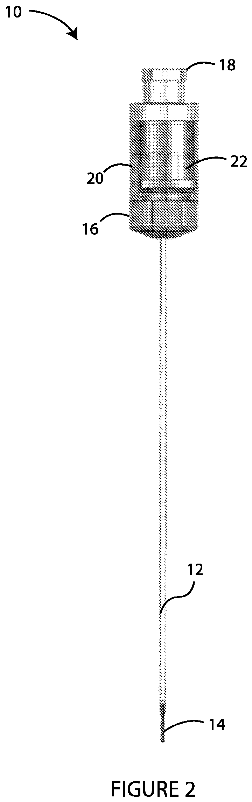

The medical instrument 10 further includes a collar 16, a fluid fitting 18, a removable clip 20, and a locking mechanism 22. The hypodermic needle 12 is rigidly connected to the collar 16 to form a hypodermic needle subassembly 24, and the stylet 14 is rigidly connected to the fluid fitting 18 to form a stylet subassembly 26. As discussed in more detail below, when the medical instrument 10 is assembled, the stylet 14 fits within the hypodermic needle 12 and the medical instrument 10 is adjustable between a retracted configuration in which the stylet is sheathed within the hypodermic needle (shown in Figure 1) and an protracted configuration in which a portion of the stylet extends from the hypodermic needle (shown in Figure 2). In addition, as discussed in more detail below, the stylet 14 is rotatable within the hypodermic needle 12.

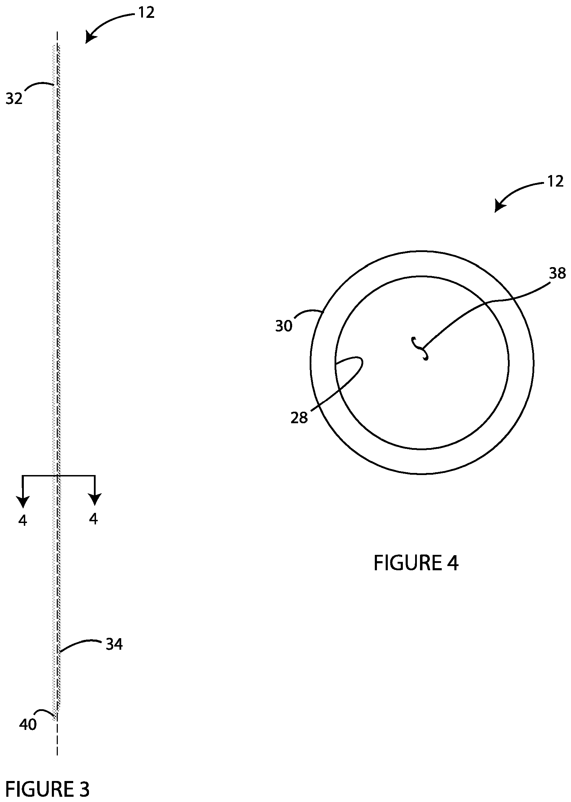

As seen in Figures 3-4, the hypodermic needle 12 has an inner needle surface 28, an outer needle surface 30, a proximal needle end 32, a distal needle end 34, and a needle axis 36. The inner needle surface 28 defines a needle bore 38 extending from the proximal needle end 32 to the distal needle end 34. The distal needle end 34 has a sharpened distal tip 40 configured to puncture soft tissue, such as the skin. The sharpened distal tip 40 enables the hypodermic needle 12 to easily puncture the skin of a patient to gain interior access to the human anatomy.

In a preferred embodiment, the hypodermic needle 12 is configured to conform to a size defined by the Birmingham gauge. The Birmingham gauge is a system used to specify thickness and/or diameter of hypodermic needles. The Birmingham gauge is also known as the Birmingham wire gauge. The following table provides outer diameter, inner diameter, and nominal wall thickness for hypodermic needles defined by the Birmingham gauge. The inner diameters and nominal wall thicknesses of the various. gauges may vary from the dimensions shown below.

| **Needle** | **Outer diameter** | | **Inner diameter** | | **Wall thickness** | |
|---|---|---|---|---|---|---|
| *Gauge* | *mm* | *tolerance (mm)* | *mm* | *tolerance (mm)* | *mm* | *tolerance (mm)* |
| 7 | 4.572 | +0.025 | 3.810 | ±0.076 | 0.381 | +0.025 |
| 8 | 4.191 | +0.075 | 3.429 | +0.076 | 0.381 | +0.025 |
| 9 | 3.759 | +0.075 | 2,997 | +0.076 | 0.381 | +0.025 |
| 10 | 3.404 | ±0.025 | 2.692 | ±0,076 | 0.356 | +0.025 |
| 11 | 3.048 | +0.025 | 2.388 | +0.076 | 0.330 | +0.025 |
| 12 | 2.769 | +0.025 | 2.159 | +0.076 | 0.305 | +0.025 |
| 13 | 2.413 | +0.025 | 1.803 | +0.076 | 0.305 | +0.025 |
| 14 | 2.108 | +0.025 | 1.600 | +0.076 | 0.254 | +0.025 |
| 15 | 1.829 | +0.013 | 1.372 | +0.038 | 0.229 | ±0.013 |
| 16 | 1.651 | +0.013 | 1.194 | +0.038 | 0.229 | +0.013 |
| 17 | 1.473 | +0.013 | 1.067 | +0.038 | 0.203 | +0.013 |
| 18 | 1.270 | +0.013 | 0.838 | +0.038 | 0.216 | ±0.013 |
| 19 | 1.067 | ±0.013 | 0.686 | ±0.038 | 0.191 | ±0.013 |
| 20 | 0.9081 | ±0.0064 | 0.603 | ±0.019 | 0.1524 | ±0.0064 |
| 21 | 0.8192 | ±0.0064 | 0.514 | ±0.019 | 0.1524 | ±0.0064 |
| 22 | 0.7176 | ±0.0064 | 0.413 | ±0.019 | 0.1524 | ±0.0064 |
| 22 | 0.7176 | ±0.0064 | 0.152. | ±0.019 | 0.2826 | ±0.0064 |
| 23 | 0.6414 | ±0.0064 | 0.337 | ±0.019 | 0.1524 | ±0.0064 |
| 24 | 0.5652 | ±0.0064 | 0.311 | ±0,019 | 0.1270 | ±0.0064 |
| 25 | 0.5144 | ±0,0064 | 0.260 | +0,019 | 0.1270 | ±0.0064 |
| 26 | 0.4636 | ±0.0064 | 0.260 | ±0.019 | 0.1016 | ±0.0064 |
| 26 | 0.4737 | ±0.0064 | 0.127 | ±0.019 | 0.1734 | ±0.0064 |
| 27 | 0.4128 | ±0.0064 | 0.210 | ±0.019 | 0.1016 | ±0.0064 |
| 28 | 0.3620 | ±0.0064 | 0.184 | ±0.019 | 0.0889 | ±0.0064 |
| 29 | 0.3366 | ±0.0064 | 0.184 | ±0.019 | 0.0762 | ±0.0064 |
| 30 | 0.3112 | ±0.0064 | 0.159 | ±0.019 | 0.0762 | ±0.0064 |
| 31 | 0.2604 | ±0.0064 | 0.133 | ±0.019 | 0.0635 | ±0.0064 |
| 32 | 0.2350 | ±0.0064 | 0.108 | ±0.019 | 0.0635 | ±0.0064 |

It is contemplated that in certain embodiments, a medical instrument in accordance with this disclosure can substantially conform to the dimensions of a gauge standard of any one of the Birmingham wire gauges listed in the chart above. A hypodermic needle can have an outer diameter in the range of outer diameters of the Birmingham gauge needles listed in the above chart and/or have an inner diameter in the range of the inner diameters of the Birmingham gauge needles listed in the above chart. Needles in the scope of this disclosure need not strictly conform to a Birmingham gauge standard in one or more embodiments. In one embodiment of the present disclosure, the outer and inner diameters of the hypodermic needle 12 are smaller than a 14-gauge needle and larger than a 28-gauge needle, as defined by the Birmingham gauge. In another embodiment, the outer and inner diameters of the hypodermic needle 12 are smaller than a 17-gauge needle and larger than a 23-gauge needle, as defined by the Birmingham gauge. In yet another embodiment, the outer and inner diameters of the hypodermic needle 12 is smaller than an 18-gauge needle and larger than a 22-gauge needle, as defined by the Birmingham gauge. The size of the hypodermic needle 12 will vary depending upon the type of interventional radiology procedure being performed and the purpose of the underlying interventional radiology procedure. For example, for interventional radiology procedures performed on hands, wrists, feet, and/or ankles, the hypodermic needle 12 will likely be a hypodermic needle smaller than a 17-gauge needle and larger than a 23-gauge needle, as defined by the Birmingham gauge. A hypodermic needle of this size enables a radiologist to perform an interventional radiology procedure within the space constraints surrounding hands, wrists, feet, and/or ankles. A person of ordinary skill in the art will understand that the hypodermic needle does not have to conform to a size defined by the Birmingham gauge.

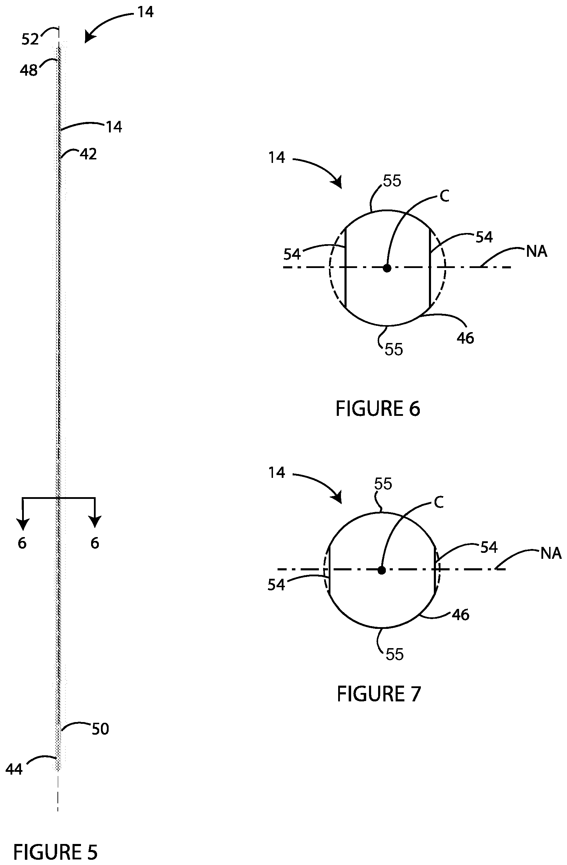

As seen in Figures 5-6, the stylet 14 has a stylet body 42, a stylet head 44, an outer stylet surface 46, a proximal stylet end 48, a distal stylet end 50, and a stylet axis 52. The stylet 14 is shaped and sized to be received within the hypodermic needle 12. In one embodiment, the stylet 14 is a solid monolithic component devoid of any bores. The outer stylet surface 46 forms a perimeter of the stylet 14.

The stylet 14 is configured such that after medical instrument 10 is assembled, the medical instrument can pass fluid through the needle bore 38 along the outer stylet surface 46 of the stylet such that fluid is discharged from the distal needle end 34. The outer stylet surface 46 of the stylet 14 includes at least one longitudinal fluid-passing surface and at least one bearing surface. In the embodiment shown in Figures 5-6, the outer stylet surface 46 of the stylet body 42 includes a pair of flats 54 oriented on diametrically opposite sides of the stylet 14 and a pair of curved surface portions 55. Each of the flats 54 constitutes a longitudinal fluid-passing surface portion, and each of the curved surface portions 55 constitutes a fluid bearing surface portion. As seen in Figures 5-6, the flats 54 are interleaved between the curved surface portions 55. A person of ordinary skill in the art will understand that there multiple ways of forming the flats 54 onto a stock of material from which the stylet 14 is manufactured. A person of ordinary skill in the art will further understand that depending upon the specific case in which the medical instrument 10 is being used and the number of longitudinal fluid-passing surfaces needed, the outer stylet surface 46 of the stylet body 42 may have only one flat and one curved surface portion, or more than two flats spaced at spaced apart locations around the stylet's perimeter and more than one curved surface portion. In an alternative embodiment, the outer stylet surface of the stylet body may include a pair of grooves oriented on diametrically opposite sides of the stylet in lieu of flats. In such an instance, each groove would constitute a longitudinal fluid-passing surface. A person of ordinary skill in the art will understand that the flats or grooves do not have to be oriented on diametrically opposite sides of the stylet.

As seen in Figure 6, the perimeter of the stylet 14 would be substantially circular in shape in cross-section (illustrated by the dashed lines) but for the flats 54. Thus, in one or more embodiments, the stylet 14 comprises cylindrical stock from which material is removed to form the flats. The stylet body 42 has a neutral axis NA and a centroid C, with the neutral axis passing through the centroid. As illustrated by Figures 6-7, the greater the distance between the centroid C and each of the flats 54, the less material removed from the stylet body 42 when the flats are being formed. The less material removed from the stylet body 42 when forming the flats 54, the greater the overall strength and stiffness of the stylet 14. At the same time, the greater the distance between the centroid C and each of the flats 54, the less fluid that can be passed through the hypodermic needle 12 during a specific time period because of smaller fluid passageways. The fluid passageways are discussed in more detail below.

The stylet head 44 is located at the distal stylet end 50 of the stylet 14. The medical instrument 10 can be used for various reasons within various types of interventional radiology procedures, depending on a number of factors (e.g., size of the hypodermic needle, type of fluid being imparted through the medical instrument, imaging method, etc.). One primary factor affecting how a radiologist will use the medical instrument 10 is the type and/or design of the stylet head 44 of the stylet 14. The stylet head 44 enables the radiologist to perform a number of functions within a patient, including cutting and/or moving soft tissue. The various designs for the stylet head 44 are discussed in more detail below.

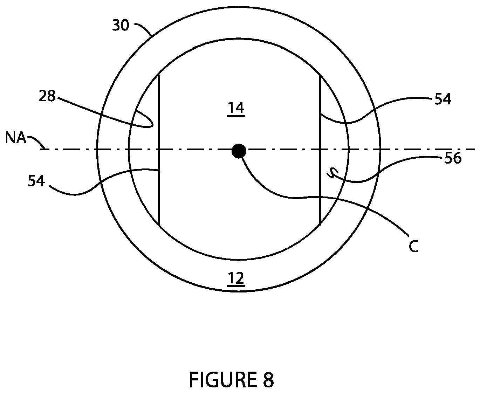

When the medical instrument 10 is assembled, which is shown Figures 1-2, the stylet 14 is received within the hypodermic needle 12 in a manner such that the stylet axis 52 is coaxial with the needle axis 36. At least a portion of the stylet 14 is located within the needle bore 38. In the embodiment shown in Figures 1-2, a substantial portion of the stylet body 42 is located within the needle bore 38. When the medical instrument 10 is assembled, the stylet 14 and the hypodermic needle 12 collectively form fluid passageways 56, as seen in Figure 8. In the embodiment shown in Figures 1-2, the medical instrument 10 includes two fluid passageways 56. Each fluid passageway 56 is formed by the inner needle surface 28 of the hypodermic needle 12 and the outer stylet surface 46 of the portion of the stylet 14 located within the needle bore 38. More specifically, each fluid passageway 56 is collectively formed by the inner needle surface 28 of the hypodermic needle 12 and the flats 54 of the stylet 14 that are located within the needle bore 38. The fluid passageways 56 enable fluid to be passed through the needle bore 38 of the hypodermic needle 12 along the stylet 14 such that fluid is discharged from the distal needle end 34. In this manner, fluid can be discharged from the distal needle end 34 even when the stylet 14 is located within the needle bore 38.

When the medical instrument 10 is assembled, the stylet 14 is movable relative to the hypodermic needle 12 along the needle axis 36. The ability of the stylet 14 to move relative to the hypodermic needle 12 along the needle axis 36 enables the medical instrument to be adjusted from the retracted configuration (shown in Figure 1) to the protracted configuration (shown in Figure 2). In addition, when the medical instrument 10 is assembled, the stylet 14 is rotatable about the needle axis 36. When the medical instrument is being adjusted from the retracted configuration to the protracted configuration or being rotated about the needle axis 36, the curved surface portions 55 of the outer stylet surface 46 bear against the inner needle surface 28 of the hypodermic needle 12. The curved surface portions 55 thus keep the stylet 14 centered in the needle bore 38 as the stylet slides and/or rotates with respect to the needle 12.

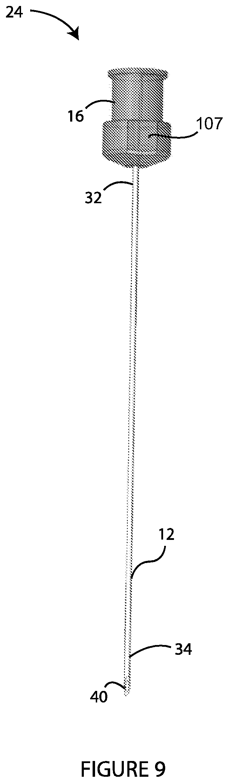

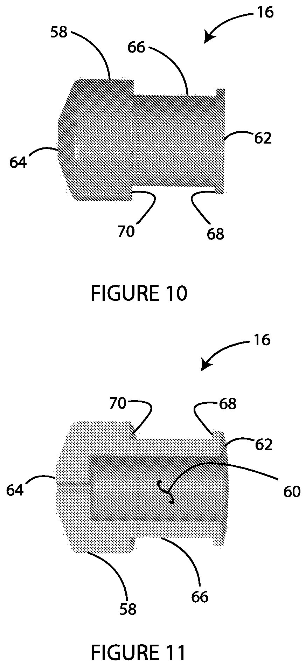

The hypodermic needle 12 is fixedly connected to the collar 16, as shown in Figure 9, forming the hypodermic needle subassembly 24. The collar 16, which is shown in Figures 10-11, includes an outer collar surface 58 and a collar bore 60 extending from a proximal collar end 62 to a distal collar end 64. The outer collar surface 58 includes a grooved region 66 having a first stop 68 and a second stop 70. The collar bore 60 is sized and configured at the distal collar end 64 to snugly receive the proximal needle end 32 of the hypodermic needle 12 and fix the hypodermic needle relative to the collar 16. In one or more embodiments, the proximal end 32 of the hypodermic needle 12 forms a friction fit or interference fit with collar 16 when it is snugly received in the collar bore 60. The proximal needle end could also be joined to the collar in other ways, such as by an adhesive bond, thermal bond, interlocking mechanical parts, etc. Suitably, the proximal needle end 32 is secured such that the needle 12 and the collar 16 are constrained to move conjointly as a unit.

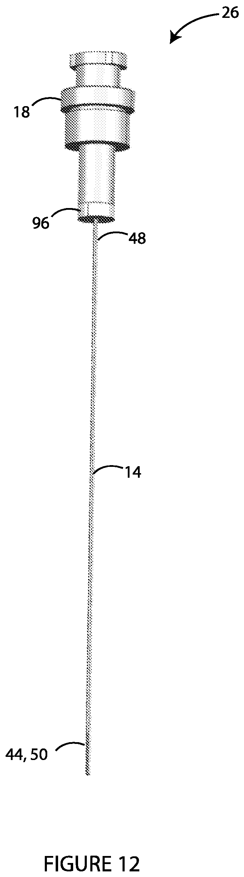

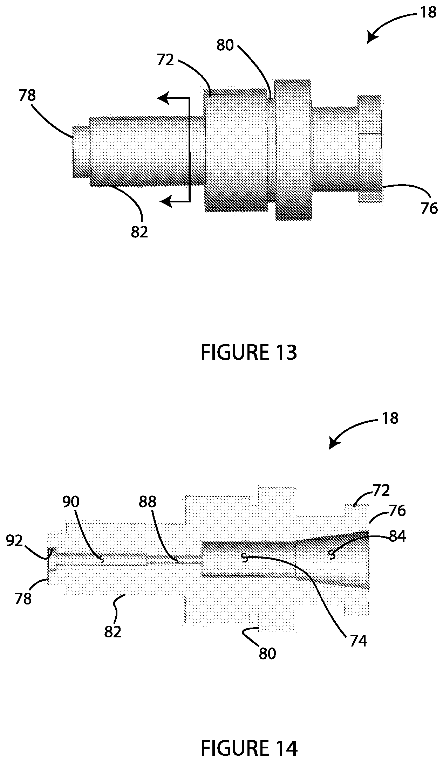

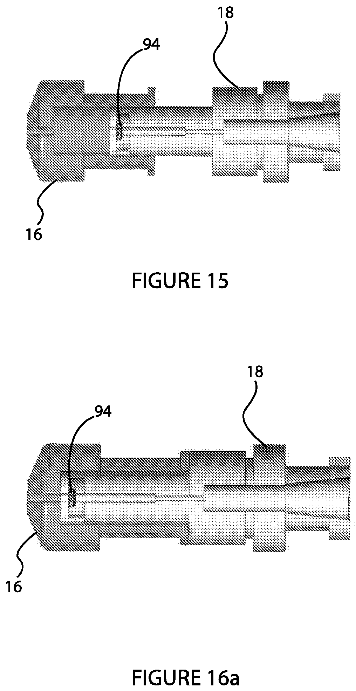

The stylet 14 is fixedly connected to a fluid fitting 18, as shown in Figure 12. The stylet 14 and the fluid fitting 18 partially form the stylet subassembly 26. In one embodiment, the fluid fitting 18 is a Luer lock fitting. In another embodiment, the fluid fitting 18 is a Luer-slip fitting. A person of ordinary skill in the art will understand and appreciate that other types of fluid fittings can be used in place of a Luer lock fitting or a Luer-slip fitting. The fluid fitting 18, which is shown in Figures 13-14, includes an outer fitting surface 72 and a fitting bore 74 extending from a proximal fitting end 76 to a distal fitting end 78. The outer fitting surface 72 of the fluid fitting has a channeled region 80 and a male region 82. The male region 82 of the fluid fitting 18 is sized and configured to fit within the collar bore 60 of the collar 16, as seen in Figures 15-16a.

As seen in Figure 14, the fitting bore 74 of the fluid fitting 18 includes a receiver region 84, a stylet region 88, a needle region 90, and a sealing channel 92. The receiver region 84 is configured to receive a syringe 86 and is located at the proximal fitting end 76 of the fitting bore 74. The stylet region 88 is downstream of the receiver region 84 and is configured to snugly receive the proximal stylet end 48 of the stylet 14, thereby fixing the stylet relative to the fluid fitting 18. In one or more embodiments, the proximal stylet end 48 (e.g., the curved bearing surface portions 55) forms a friction fit or interference fit with stylet region 88. The proximal stylet end could also be joined to the fluid fitting in other ways, such as by an adhesive bond, thermal bond, interlocking mechanical parts, etc. Suitably, the proximal stylet end 48 is secured such that the stylet 14 and the fluid fitting 18 are constrained to move conjointly as a unit.

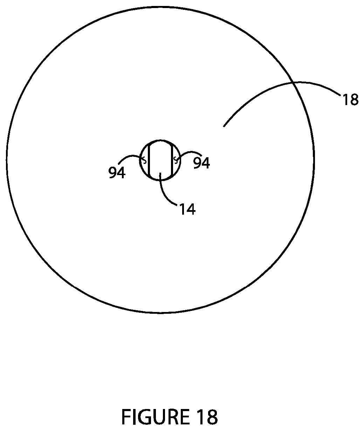

Further, the proximal stylet end 48 is secured to the fluid fitting 18 such that fluid is passable through the interface between the proximal stylet end and the fluid fitting in one or more embodiments. For example, in the illustrated embodiment, the stylet region 88 is substantially circular in cross-section. Thus, although the stylet region 88 snugly receives the proximal stylet end 48 of the stylet 14, fluid channels 94 are formed between the fluid fitting 18 and the stylet because of the flats 54, as seen in Figure 18.

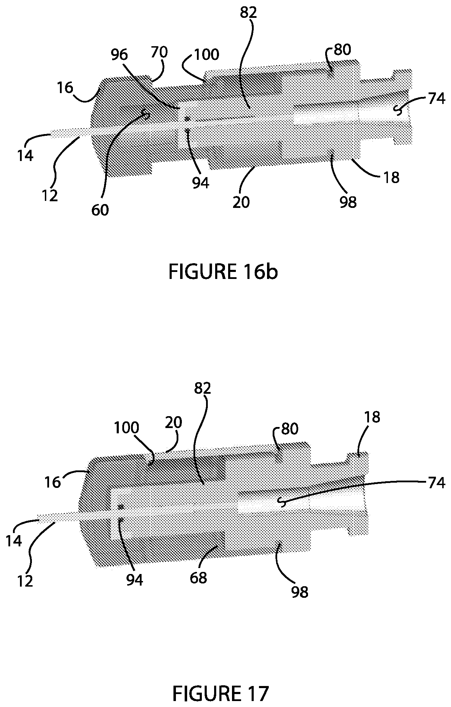

As seen in Figure 14, the needle region 90 is downstream of the stylet region 88 and is configured to accommodate movement of the proximal needle end 32 of the hypodermic needle 12. As the medical instrument 10 adjusts from the retracted configuration to the protracted configuration and vice versa, the proximal needle end 32 of the hypodermic needle 12 will move axially within the needle region 90. This can be seen in Figures 16b-17. The sealing channel 92 is downstream of the needle region and is configured to accommodate a seal 94 (e.g., an 0-ring). The seal 94 fits snugly within the sealing channel 92 and is sized to form a fluid tight seal between the fluid fitting 18 and the outer needle surface 30 when the medical instrument 10 is assembled. The seal 94 is slidably and sealingly engaged with the outer needle surface 30 such that the fluid seal is maintained as the fluid fitting 18 moves axially with the stylet 14 with respect to the needle 12. A ferrule 96 can be attached or adhered to the distal fitting end 78 to ensure the seal 94 remains within the sealing channel 92 as the hypodermic needle 12 moves axially relative to the seal. Generally, it can be seen that the fluid fitting 18, needle 12, seal 94 and stylet 14 define passaging that provides sealed fluid communication between a syringe 86 and the passages 56 defined between the inner needle surface 28 and the stylet flats 54. As such, during use of the medical instrument 10, fluid can be directed from a syringe 86 through the needle bore 38 and along the stylet 14 so that the fluid can be discharged from the distal needle end.

Accordingly, the stylet 14, the fluid fitting 18, the seal 94, and the ferrule 96 are fixed relative to each other and collectively form the stylet subassembly 26.

As seen in Figures 1-2, when the medical instrument 10 is assembled, the stylet subassembly 26 is connected to the hypodermic needle subassembly 24 by the removable clip 20. As shown in Figures 16b-17, the removable clip 20 has a proximal ledge 98 and a distal ledge 100. The proximal ledge 98 of the removable clip 20 snaps within the channeled region 80 of the fluid fitting 18, thereby fixing the removable clip 20 relative to the fluid fitting. The distal ledge 100 of the removable clip 20 fits within the grooved region 66 of the collar 16 in a manner such that the distal ledge of the removable clip can move axially between the first and second stops 68, 70 of the collar. When the stylet subassembly 26 is connected to the hypodermic needle subassembly 24 via the removable clip 20, the stylet 14 can rotate about the stylet axis 52 (which is coaxial with the needle axis 36) within the needle bore 38 of the hypodermic needle 12. Additionally, the stylet subassembly 26 can move axially relative to the hypodermic needle subassembly 24, thereby enabling the medical instrument 10 to adjust from the retracted configuration to the protracted configuration. In other words, as the distal ledge 100 of the removable clip 20 slides between the first and second stops 68, 70 of the collar 16, the male region 82 of the fluid fitting 18 can move axially within the collar bore 60 of the collar 16. Similarly, the proximal needle end 32 of the hypodermic needle 12 can move axially within the needle region 90 of the fluid fitting 18.

As seen in Figure 16b, when the medical instrument 10 is in the retracted configuration, the distal ledge 100 of the removable clip 20 is adjacent the first stop 68 of the collar 16. When the medical instrument 10 is in the retracted configuration, the stylet 14 is in a retracted position in which the stylet head 44 is located within the needle bore 38 (as seen in Figure 1). As seen in Figure 17, when the medical instrument 10 is in the protracted configuration, the distal ledge of the removable clip is adjacent the second stop 70 of the collar 16. When the medical instrument 10 is in the retracted configuration, the stylet 14 is in a protracted position in which the stylet head 44 is located at least partially external to the needle bore 38 (as seen in Figure 2). A person of ordinary skill in the art will understand that a number of factors may determine how much of the stylet head 44 is external to the needle bore 38. For example, the amount of the stylet 14 extending in a distal direction from the needle bore 38 will vary depending upon the design of the stylet head 44 and/or the type of interventional radiology procedure being performed.

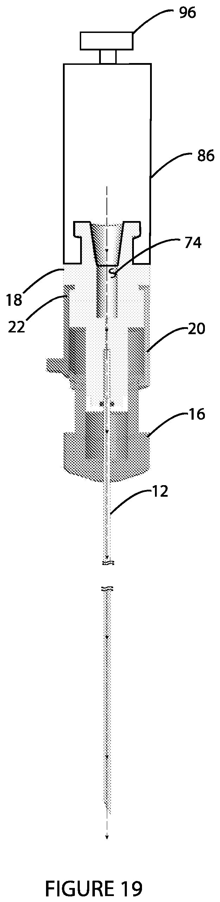

As seen in Figure 19, the receiver region 84 of the fluid fitting 18 is configured to receive syringe 86. Figure 19 generally shows the flow of fluid in the medical instrument 10, with the stylet 14 removed from the image to better illustrate fluid flow. Syringe 86 includes an interior volume containing fluid (e.g., lidocaine or saline or a combination thereof). At the start of an interventional radiology procedure, syringe 86 is received within the receiver region 84 of the fluid fitting 18. When syringe 86 is received within the syringe receiver 84, fluid within the interior volume of the syringe is fluidly connected with the fitting bore 74. In other words, fluid from the interior volume of syringe 86 can pass through the fitting bore 74. Fluid from the interior volume of syringe 86 is forced from the interior volume and into the fitting bore 74 as a syringe plunger 96 of the syringe is depressed. Fluid from the syringe 86 passes through the receiver region 84 of the fluid fitting 18 into the stylet region 88. Fluid subsequently flows through the fluid channels 94 formed between the fluid fitting 18 and the flats 54 of the stylet 14 and into the needle region 90. Regardless of the position of the proximal needle end 32 within the needle region 90, fluid is forced into the fluid passageways 56 formed by the inner needle surface 28 of the hypodermic needle 12 and the flats 54 of the stylet 14 located within the needle bore 38. Seal 94 ensures fluid is forced into the fluid passageways 56.

Fluid then flows along the stylet body 42 from the proximal needle end 32 to the distal needle end 34 through fluid passageways 56. Fluid originating from the interior volume of syringe 86 is ultimately discharged from the medical instrument 10 through the sharpened distal tip 40 of the hypodermic needle 12. Notably, fluid from the syringe 86 follows this general fluid path regardless of whether the medical instrument 10 is in the retracted configuration, the protracted configuration, or any intermediate configuration. Accordingly, during an interventional radiology procedure, fluid from the syringe 86 can be injected continuously or intermittently regardless of positioning of the stylet 14 within the hypodermic needle 12. As discussed in more detail below, the ability to inject fluid at any point during an interventional radiology procedure enables a radiologist to more readily identify the position and/or movement of soft tissue for certain imaging methods (e.g., ultrasound). Additionally, the ability to inject fluid at any point during an interventional radiology procedure enables a radiologist to hydrodissect soft tissue throughout the procedure, as discussed in more detail below.

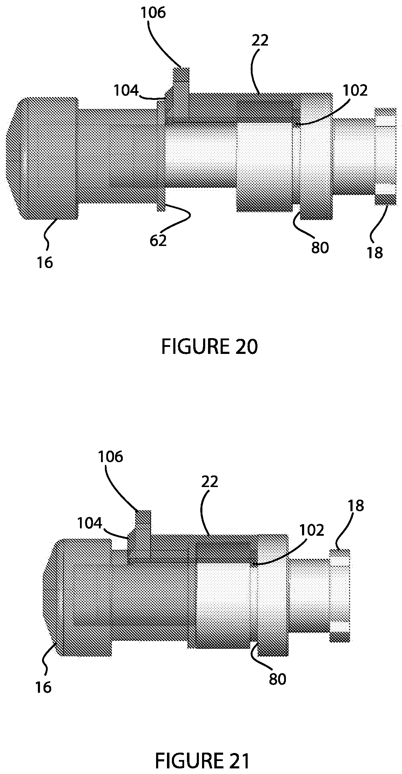

The medical instrument 10 may further include a locking mechanism 22, which can be seen in Figures 20-21. When initiated, the locking mechanism 22 prevents the stylet subassembly 26 from moving relative to the needle subassembly 24. In this manner, the locking mechanism 22 prevents the medical instrument 10 from adjusting from the retracted configuration to the protracted configuration when initiated. The locking mechanism 22 includes a proximal locking ledge 102, a distal locking ledge 104, and a pull tab 106. The proximal locking ledge 102 snaps within the channeled region 80 of the fluid fitting 18, thereby fixing the locking mechanism 22 relative to the fluid fitting. The locking mechanism 22 is designed such that when engaged, the distal locking ledge 104 abuts the proximal collar end 62 of the collar 16 and prevents movement of the stylet subassembly 26 relative to the needle subassembly 24. In the locked configuration, additional structure associated with the collar 16 can also engage the proximal end of the ledge 102 in one or more embodiments. Further, structure associated with the collar 16 can engage one or both sides of the ledge 102. In this way, the locking mechanism can be configured to inhibit movement of the fluid fitting relative to the collar in the distal, proximal, and/or rotational directions when the locking mechanism is locked.

To release the locking mechanism 22, the radiologist pulls the pull tab 106 outwardly such that the distal locking ledge 104 no longer abuts the proximal collar end 62. This enables the distal locking ledge 104 to slide past the proximal collar end 62, thus enabling the stylet subassembly 26 to move relative to the needle subassembly 24. In this manner, the locking mechanism 22 can be released to enable the medical instrument 10 to adjust from the retracted configuration (shown in Figure 20) to the protracted configuration (shown in Figure 21). A person of ordinary skill in the art will understand that the locking mechanism 22 may be a one-piece component made of a suitable material that enables a radiologist to readily release the locking mechanism by pulling the pull tab 106 while still ensuring that the locking mechanism prevents movement of the stylet subassembly 26 relative to the needle subassembly 24 when the locking mechanism is engaged.

A person of ordinary skill in the art will understand and appreciate that other types of locking mechanisms could be used for the medical instrument 10 that do not incorporate a pull tab. For example, the medical instrument 10 could be designed to incorporate a twist type locking mechanism. In such an embodiment, the removable clip 20 and the collar 16 could be keyed in a manner such that the removable clip (and therefore the stylet subassembly 26) cannot be moved axially relative to the needle subassembly 24 until rotating the removable clip and the stylet subassembly. Additional alternative locking mechanisms for the medical instrument 10 include, but are not limited to, collets, set screws, removable snap-in blocks and keys, and any manner of other devices. These locking mechanisms could be used to lock linear motion of the stylet subassembly 26 relative to needle assembly 24. In addition or alternatively, these locking mechanisms could be used to lock rotational movement of the stylet subassembly 26 about the stylet axis 52.

A person of ordinary skill in the art will further understand and appreciate that various components of the medical instrument 10 can be designed in a manner to provide an extracorporeal indicator to the radiologist of the orientation of the hypodermic needle 12 (e.g., bevel up or bevel down) and the orientation of the stylet 14. For example, as seen in Figure 9, the collar 16 includes a flattened region 107 that corresponds to a position of a bevel of the sharpened distal tip 40. Thus, the flattened region 107 of the collar 16 provides an extracorporeal indicator to the radiologist of the orientation of the hypodermic needle 12. The radiologist will also be able to determine the orientation of the hypodermic needle 12 via the imaging method being used for the interventional radiology procedure. Additionally, the pull tab 106 of the locking mechanism 22 can be used to provide the radiologist an extracorporeal indicator of the orientation of the stylet 14. As seen in Figure 2, a projection of the pull tab 106 corresponds to a position of the stylet head 44, thereby enabling the radiologist to determine the orientation of the stylet head. This is particularly helpful when the stylet 14 is in the retracted position and sheathed by the hypodermic needle 12. After the stylet 14 is in the protracted position, the radiologist will also be able to determine the orientation of the stylet head 44 via the imaging method being used for the interventional radiology procedure.

In general, one or both of the collar 16 and the fluid fitting 18 forms a handle that is gripped by the radiologist and manipulated by hand to control the medical instrument 10. In addition to the handle formed by the collar and/or fitting, a radiologist may grip and manipulate the instrument 10 using the syringe 86 (broadly, fluid source) that is coupled to the fluid fitting 18. When the components at the proximal end portion of the medical instrument 10 are considered to constitute a handle, it is apparent that the collar 16 generally forms a handle housing and the fluid fitting 18 generally forms a carriage having a portion that slidably received in the housing for movement along the needle axis with respect to the housing. Further, in the illustrated embodiment, the carriage (fluid fitting 18) is rotatably received in the housing (collar 16) for rotation with respect to the housing about the needle axis. It will be understood that other handle configurations can be used for the medical instrument. In general, in a suitable handle, one of the housing and the carriage can comprise a fluid coupling configured to couple the medical instrument to a fluid source, and together the housing and carriage can define passaging providing sealed fluid communication between the fitting and the needle bore. In exemplary embodiments of handles within the scope of this disclosure, the carriage (e.g., the fitting 18) is movable relative to the housing (e.g., the collar 16) through a range of motion comprising a proximal end position and a distal end position. Further, certain handles within the scope of this disclosure include one or more locking mechanisms configured to selectively and releasably lock the carriage at one or both of the proximal end position and the distal end position in the range of motion.

### Stylet Heads

The medical instrument 10 can be used for a variety of reasons in various types of interventional radiology procedures, depending on a number of factors (e.g., size of the hypodermic needle, type of fluid being pushed through the medical instrument, imaging method, etc.). One factor is the type and/or design of the stylet head 44 of the stylet 14. As an example, with one type of a stylet head, the medical instrument 10 can be used to cut soft tissue for performing a carpal tunnel release guided by one type of an imaging method (e.g., ultrasound). With another type of a stylet head, the medical instrument 10 can be used to cut soft tissue for performing a De Quervain's tendon release guided by another type of an imaging method (e.g., MRI). Yet, with a third type of a stylet head, the medical instrument 10 can be used to move soft tissue to enable a prostate biopsy to be performed while using an imaging method. Accordingly, a person of ordinary skill in the art will understand that the medical instrument in accordance with the present disclosure is extremely versatile and capable of being used for multiple types of interventional radiology procedures.

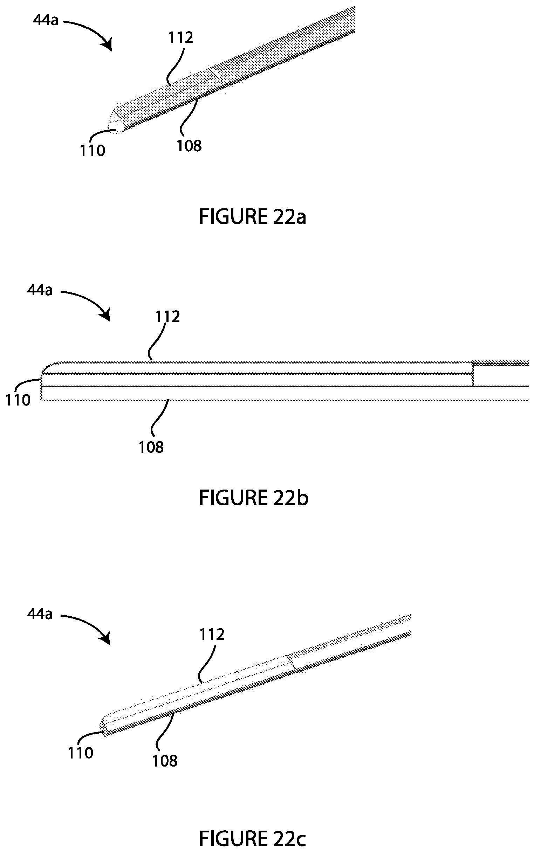

Figures 22a-22c show a first design of a stylet head 44a located at the distal stylet end 50 of the stylet 14. The stylet head 44a has a curved surface 108, an atraumatic distal tip 110, and a cutting edge 112 (broadly, a cutting element). The atraumatic distal tip 110 is configured such that the tip can move soft tissue without damaging or cutting the soft tissue. This ensures that the atraumatic distal tip 110 does not cut any soft tissue as the medical instrument 10 moves from the retracted configuration to the protracted configuration. The curved surface 108, which is convex in this embodiment and opposite the cutting edge 112, is also atraumatic. The curved surface 108 forms an atraumatic region of the stylet head 44a that can be used to manipulate tissue without damaging it. Thus in the illustrated embodiment, the stylet head 44a comprises a cutting edge 112 that is spaced apart about the perimeter of the stylet head from the atraumatic region 108. This allows the radiologist to select between cutting and atraumatically moving tissue by simply rotating the stylet until the desired side of the stylet head 44a faces the target tissue. The cutting edge 112 comprises an edge defined by a pair of converging bevels or tapering surfaces. The cutting edge 112 is sharpened such that the cutting edge can cut soft tissue. In the illustrated embodiment, the cutting edge 112 extends longitudinally along the stylet head 44a and faces generally radially outwardly. In this manner, the stylet head 44a is designed to cut only soft tissue abutting the cutting edge 112.

The radiologist can cut soft tissue abutting the cutting edge 112 in a number of ways. For example, the radiologist can cut soft tissue using the cutting edge 112 by adjusting the medical instrument 10 from the retracted configuration to the protracted configuration or vice versa. For example, it is contemplated that tissue could be cut as the stylet is reciprocated along the needle between the retracted and protracted positions. Alternatively, the radiologist can cut soft tissue using the cutting edge 112 by placing and holding the stylet 14 in the protracted configuration and moving the entire medical instrument 10 (including the hypodermic needle 12 and the stylet) as a unit in a proximal/distal direction and/or a superficial/deep direction, thereby cutting soft tissue. Depending on the orientation of the stylet 14 within the hypodermic needle 12, the stylet may need to be rotated about the stylet axis 52 to place the cutting edge 112 adjacent soft tissue desired to be cut. In one or more embodiments, the protracted stylet head 44a is used to cut tissue by urging the cutting edge 112 toward the tissue. When the cutting edge 112 is in contact with the tissue, the radiologist can urge the stylet head outward while sliding the longitudinal cutting edge along the tissue, thereby slicing through tissue with the cutting edge. A person of ordinary skill in the art will understand that the foregoing list of examples of how a radiologist can cut soft tissue using stylet head 44a is not exhaustive.

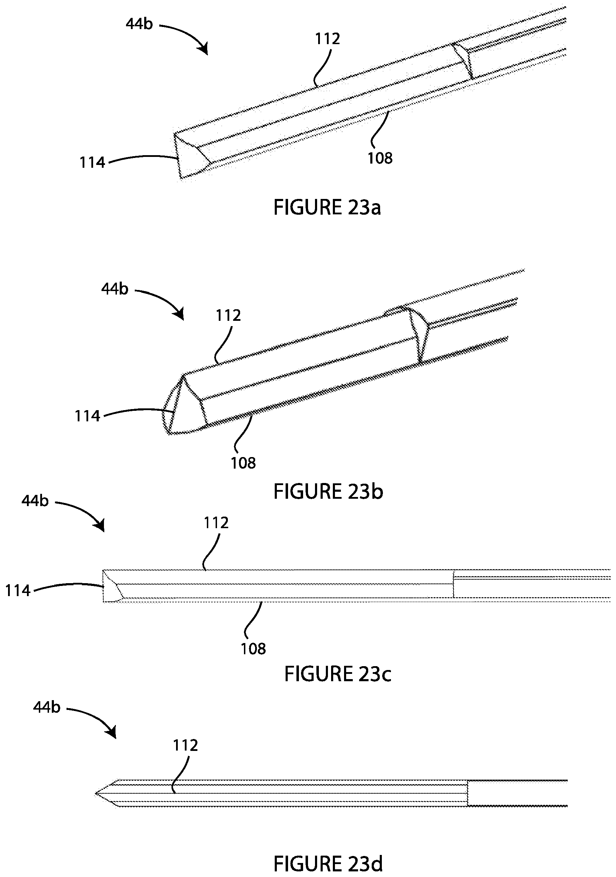

Figures 23a-23d show a second design of a stylet head 44b located at the distal stylet end 50 of the stylet 14. The stylet head 44b is similar to the stylet head 44a shown in Figures 22a-22c in that it includes a curved surface 108 and a longitudinal cutting edge 112. However, in lieu of an atraumatic distal tip, the stylet head 44b has a transverse distal cutting edge 114. Like longitudinal cutting edge 112, the distal cutting edge 114 is sharpened such that the cutting edge can cut soft tissue. Thus, for stylet head 44b, the radiologist can cut soft tissue abutting the longitudinal cutting edge 112 and soft tissue abutting the distal cutting edge 114. The radiologist can cut soft tissue in a number of ways using stylet head 44b. For example, the radiologist can cut soft tissue using the cutting edge 112 in the same manner as the longitudinal cutting edge of the stylet head 44a described above. The radiologist can also cut soft tissue using the distal cutting edge 114 by adjusting the medical instrument 10 from the retracted configuration to the protracted configuration. As yet another alternative, the radiologist can cut soft tissue using the distal cutting edge 114 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. Still further, the radiologist could attempt to slice the tissue using the distal cutting edge 114 by urging the distal cutting edge toward the tissue and sliding the cutting edge along the tissue in a direction generally parallel to the cutting edge. A person of ordinary skill in the art will understand that the foregoing list of examples of how a radiologist can cut soft tissue using stylet head 44b is not exhaustive.

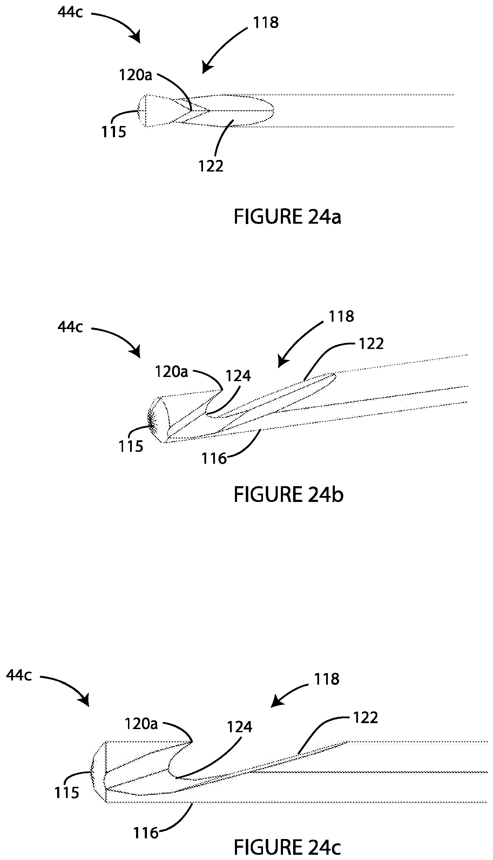

Figures 24a-24c show a third alternative design of a stylet head 44c located at the distal stylet end 50b of the stylet 14b. The stylet head 44c has an atraumatic distal tip 115, a curved surface 116, and a hook region 118 defined by a side recess formed in the stylet head. The hook region 118 includes a sharpened hook tip 120a, a shank 122, and a cutting edge 124 that faces generally proximally in the illustrated embodiment. The hook region 118 is designed such that the sharpened hook tip 120a overhangs the cutting edge 124. The overhanging nature of the sharpened hook tip 120a enables the radiologist to readily see, through an imaging method (e.g., ultrasound), the soft tissue that will be cut by the cutting edge 124. The sharpened hook tip 120a is sharpened to a point such that the hook tip can cut through or penetrate soft tissue. The shank 122 is configured to guide soft tissue toward the cutting edge 124. The cutting edge 124 is sharpened such that the cutting edge can cut soft tissue. The atraumatic distal tip 115 of the stylet head 44b is configured such that atraumatic distal tip can move soft tissue without damaging or cutting the soft tissue. This ensures that the atraumatic distal tip 115 will not damage or cut any soft tissue when the medical instrument 10 moves from the retracted configuration to the protracted configuration. The curved surface 116, which is convex in this embodiment and opposite the hook region 118, is also atraumatic such that the curved surface can move soft tissue without damaging or cutting the soft tissue. In this manner, the stylet head 44c is designed to cut only soft tissue hooked by the hook region 118 as the medical instrument 10. The radiologist can cut soft tissue abutting the cutting edge 124 in a number of ways. For example, the radiologist can cut soft tissue using the cutting edge 124 by adjusting the medical instrument 10 from the protracted configuration to the retracted configuration. Alternatively, the radiologist can cut soft tissue using the cutting edge 124 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. Depending on the orientation of the stylet 14 within the hypodermic needle 12, the stylet may need to be rotated about the stylet axis 52 to enable soft tissue to be hooked by the hook region 118 and subsequently cut by the cutting edge 124. In one or more embodiments, the protracted stylet head 44c is used by gathering tissue in the hook region 118 and guiding it along the shank 122 toward the cutting edge 124. The stylet 14 is then moved proximally relative to the tissue to draw the cutting edge through the hooked tissue, thereby cutting the tissue. In certain embodiments, the stylet 14 can also be retracted after hooking tissue in the hooked region. The inner distal edge of the needle 12 can shear through the hooked tissue as the hook region 118 draws the tissue into the needle bore 38. A person of ordinary skill in the art will understand that the foregoing list of examples of how a radiologist can cut soft tissue using stylet head 44c is not exhaustive.

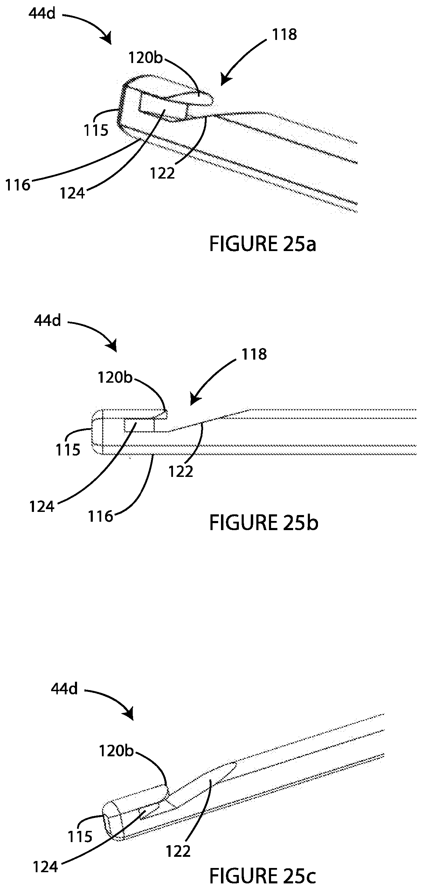

Figures 25a-25c show a fourth alternative design of a stylet head 44d located at the distal stylet end 50 of the stylet 14. The stylet head 44d is similar to the stylet head 44c shown in Figures 24a-24c in that it includes the atraumatic distal tip 115, the curved surface 116, and the hook region 118. However, in lieu of a sharpened hook tip within the hook region 118, the stylet head 44d has an atraumatic hook tip 120b. The atraumatic hook tip 120b is atraumatic such that the hook tip cannot cut through or penetrate soft tissue. As in stylet head 44c, the shank 122 is designed in a slanted manner such that the shank helps guide soft tissue towards the cutting edge 124. The radiologist can use stylet head 44d in a manner similar to that described with regard to stylet head 44c.

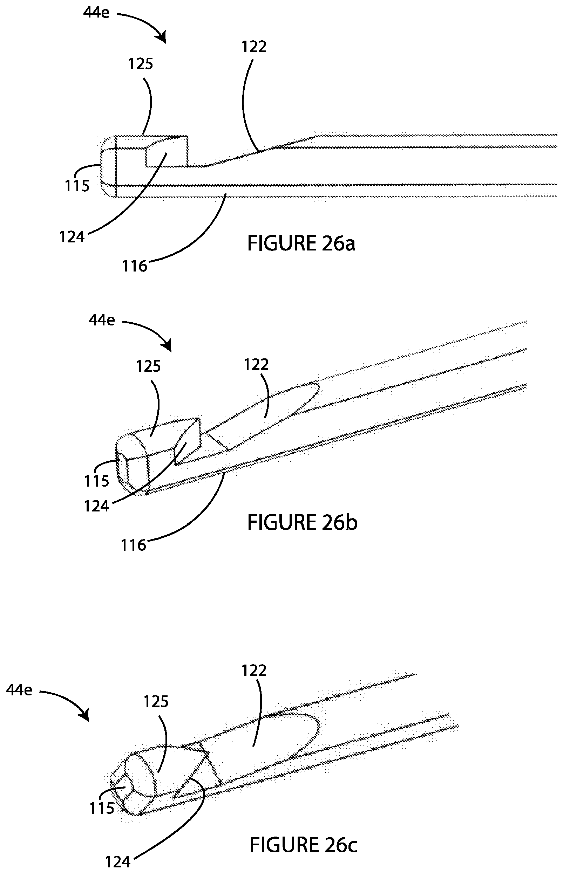

Figures 26a-26c show yet another alternative design of a stylet head 44e located at the distal stylet end 50 of the stylet 14. The stylet head 44e is similar to stylet head 44c shown in Figures 24a-24c and stylet head 44d shown in Figures 25a-25c in that it includes the atraumatic distal tip 115, the curved surface 116, and the hook region 118. However, unlike stylet head 44c (which has a sharpened hook tip) and stylet head 44d (which has an atraumatic hook tip), stylet head 44e does not include an overhanging hook tip. Instead, the cutting edge 124 extends up to a top surface 125. Stylet head 44d includes the shank 122 designed in a manner such that the shank helps guide soft tissue towards the cutting edge 124. The radiologist can cut soft tissue abutting the cutting edge 124 in a number of ways. For example, the radiologist can cut soft tissue using the cutting edge 124 by adjusting the medical instrument 10 from the protracted configuration to the retracted configuration. Alternatively, the radiologist can cut soft tissue using the cutting edge 124 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. Depending on the orientation of the stylet 14 within the hypodermic needle 12, the stylet may need to be rotated about the stylet axis 52 to enable soft tissue to be hooked by the hook region 118 and subsequently cut by the cutting edge 124. As explained above in reference to the stylet head 44c, the radiologist can also draw tissue distally along the shank 122 toward the cutting edge 124 and then urge the cutting edge through the hooked tissue to cut the tissue. A person of ordinary skill in the art will understand that the foregoing list of examples of how a radiologist can cut soft tissue using stylet head 44e is not exhaustive.

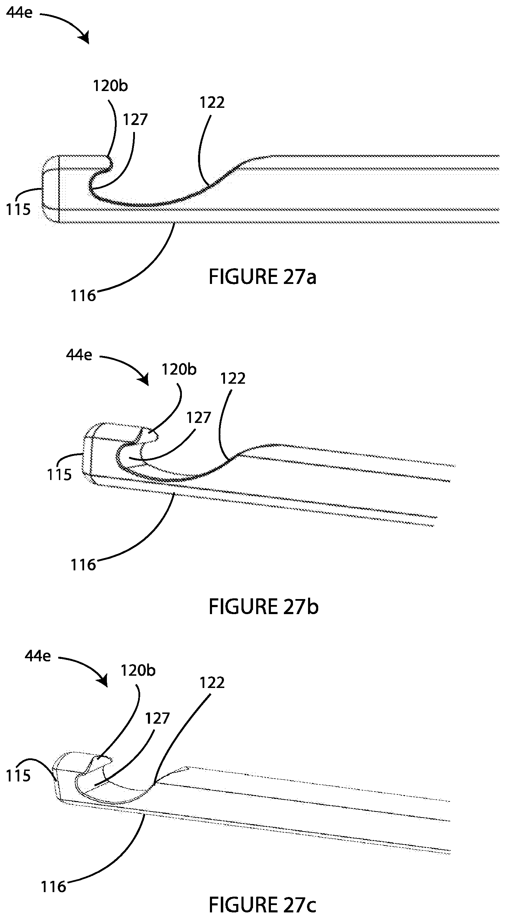

Figures 27a-27c show another alternative design of a stylet head 44f located at the distal stylet end 50 of the stylet 14. Unlike stylet heads 44a-44e, stylet head 44f does not include a cutting edge. Instead, stylet head 44f is designed to enable a radiologist to move soft tissue without damaging or cutting the soft tissue. Stylet head 44f includes the atraumatic distal tip 115, the curved surface 116, and the hook region 118. The hook region 118 includes the shank 122, the atraumatic hook tip 120b, and a throat 127. The atraumatic hook tip 120b is atraumatic such that the hook tip can move soft tissue without cutting or damaging it. Different from stylet head 44c and stylet head 44d, the hook region 118 is not designed to cut soft tissue. Instead, the hook region 118 is designed to move soft tissue hooked within the throat 127 without cutting it. Thus, the stylet head 44f is designed to enable the radiologist to hook and move soft tissue using the stylet 14. A person of ordinary skill in the art will understand that the stylet head 44f can be used in a variety of ways by a radiologist during an interventional radiology procedure.

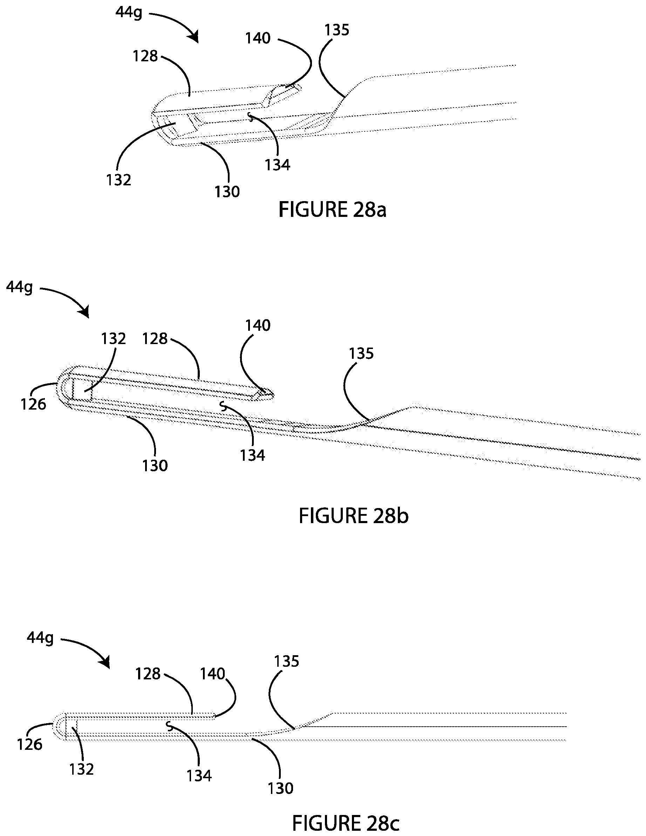

Figures 28a-28c show another alternative design of a stylet head 44g located at the distal stylet end 50 of the stylet 14. The stylet head 44g has an atraumatic distal tip 126, an upper member 128, a lower member 130, and a cutting edge 132. As used in the context of this embodiment, a person of ordinary skill in the art would understand that the terms "upper" and "lower" are interchangeable because the stylet 14 is rotatable about the stylet axis 52. The upper member 128 is spaced from the lower member 130, thereby forming a channel 134. The upper member 128 is shorter in length than the lower member 130 to enable soft tissue to enter into the channel 134. A shank 135 helps guide soft tissue towards the channel 134. The upper member 128 has a curved outer surface with chamfered edges and the lower member 130 has a curved outer surface with chamfered edges. The curved outer surface and chamfered edges of the upper and lower members 128, 130 form atraumatic surface regions. The atraumatic nature of the upper and lower members 128, 130 facilitates using the stylet head 44g to move the tissue without cutting or damaging it. The upper member 128 has a member end 140 that is atraumatic in the embodiment shown in Figures 28a-28c such that the member end is unable to cut or pierce soft tissue. A person of ordinary skill in the art will understand that in an alternative embodiment of the stylet head 44g, the member end 128 could be sharpened to a point such that it can cut or pierce soft tissue. The cutting edge 132 is located at a distal end of the channel 134 and is sharpened such that it can cut soft tissue. The atraumatic distal tip 126 of the stylet head 44g is configured such that distal tip cannot damage or cut soft tissue. This ensures that the atraumatic distal tip 126 will not cut any soft tissue when the medical instrument 10 moves from the retracted configuration to the protracted configuration. The curved outer surfaces, which are convex in this embodiment, are configured such that they cannot damage or cut soft tissue. In this manner, the stylet head 44g shown in Figures 28a-28c is designed to cut only soft tissue located within the - channel 134. The radiologist can cut soft tissue within the channel 134 in a number of ways. For example, the radiologist can gather soft tissue in the channel while the styled is protracted and then adjust the medical instrument 10 from the protracted configuration to the retracted configuration, thereby causing soft tissue located within the channel 134 to traverse distally within the channel until it is cut by the cutting edge 132 located at the distal end of the channel. Alternatively, the radiologist can cause soft tissue to traverse distally within the channel 134 by placing and holding the stylet 14 in the protracted configuration and moving the entire medical instrument 10 (including the hypodermic needle 12 and the stylet) in a proximal/distal direction and/or a superficial/deep direction, thereby causing soft tissue within the channel to be cut by cutting edge 132. Depending on the orientation of the stylet 14 within the hypodermic needle 12, the stylet may need to be rotated about the stylet axis 52 to enable soft tissue to be located within the channel 134. A person of ordinary skill in the art will understand that the foregoing list of examples of how a radiologist can cut soft tissue using stylet head 44g is not exhaustive.

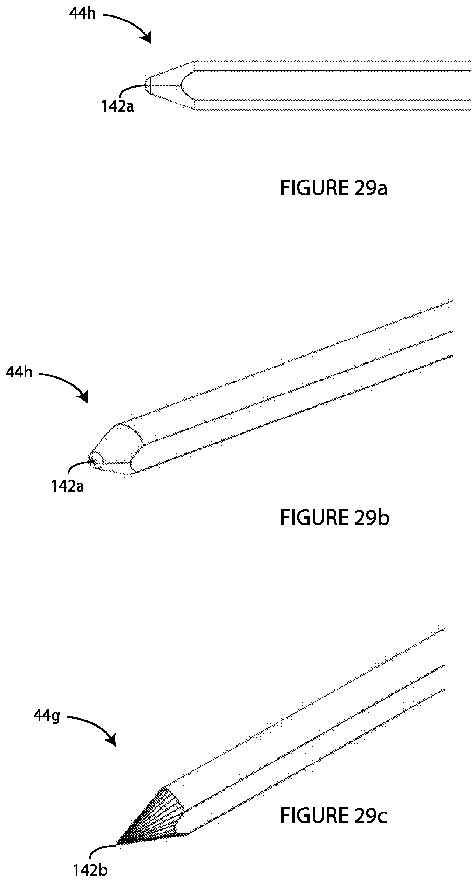

Figures 29a and 29b show another alternative design of a stylet head 44h. Stylet head 44h includes a distal point 142a. In the embodiment shown in Figures 29a and 29b, the distal point 142a is atraumatic so as to enable a radiologist to move soft tissue without damaging or cutting the soft tissue. A person of ordinary skill in the art will understand that the stylet head 44h with the atraumatic distal point 142a can be used in a variety of ways by a radiologist during an interventional radiology procedure. Figure 29c shows an alternative embodiment of the stylet head 44h. In this alternative embodiment, the distal point 142b is sharpened to a point. The sharpened distal point 142b enables the radiologist to readily pierce or puncture soft tissue when stylet 14 is moved in a proximal/distal direction and/or a superficial/deep direction. A person of ordinary skill in the art will understand that the stylet head 44h with the sharpened distal point 142b can be used in a variety of ways by a radiologist during an interventional radiology procedure.

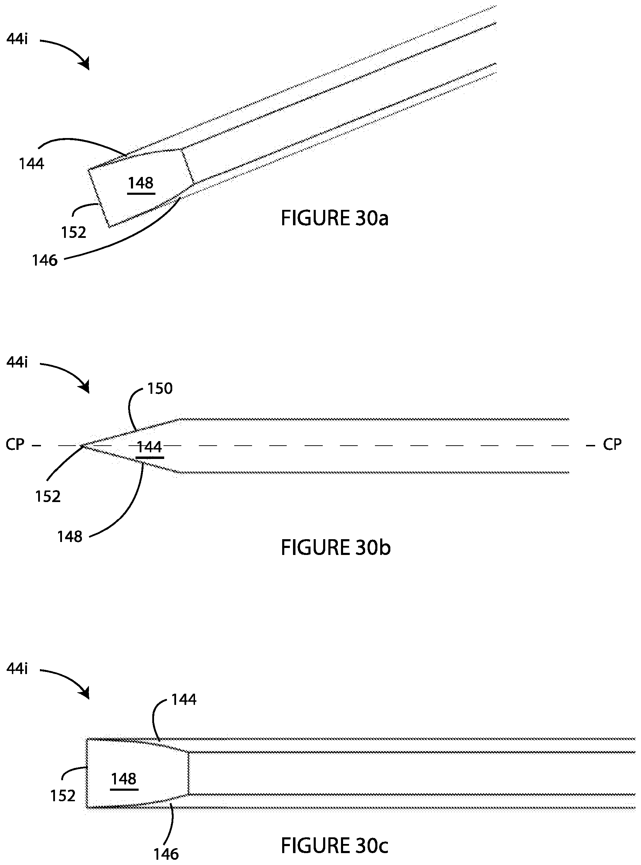

Figures 30a-30c show another alternative design of a stylet head 44i located at the distal stylet end 50 of the stylet 14. The stylet head 44i has a top curved surface 144, a bottom curved surface 146, a first forging surface 148 (e.g., a first bevel), and a second forging surface 150 (e.g., a second bevel). The first and second forging surfaces 148, 150 intersect each other in a manner that collectively forms a cutting edge 152. The top and bottom curved surfaces 144, 146, which are convex in this embodiment, are atraumatic such that the top and bottom curved surfaces cannot damage or cut soft tissue. As seen in Figure 30b, the first and second forging surfaces 148, 150 are symmetrical about a central plane CP. In this manner, the cutting edge 152 formed by the first and second forging surfaces 148, 150 is oriented along the central plane CP. The stylet head 44i is designed to cut only soft tissue abutting the cutting edge 152. The radiologist can cut soft tissue using the cutting edge 152 in a number of ways. For example, the radiologist can cut soft tissue using the cutting edge 152 by adjusting the medical instrument 10 from the retracted configuration to the protracted configuration. Alternatively, the radiologist can cut soft tissue using the cutting edge 152 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. In one or more embodiments, the protracted stylet head 44i is used to cut tissue by urging the cutting edge 152 toward the tissue and then sliding the cutting edge along the tissue, thereby slicing through tissue with the cutting edge. The distal end portion of the illustrated stylet head 44i is also wedge-shaped. It is contemplated that a radiologist could use the wedge-shaped head 44i to cleave tissue and or wedge tissue away from an adjacent structure. Depending on the orientation of the stylet 14 within the hypodermic needle 12, the stylet may need to be rotated about the stylet axis 52. A person of ordinary skill in the art will understand that the foregoing list of examples of how a radiologist can cut soft tissue using stylet head 44i is not exhaustive.

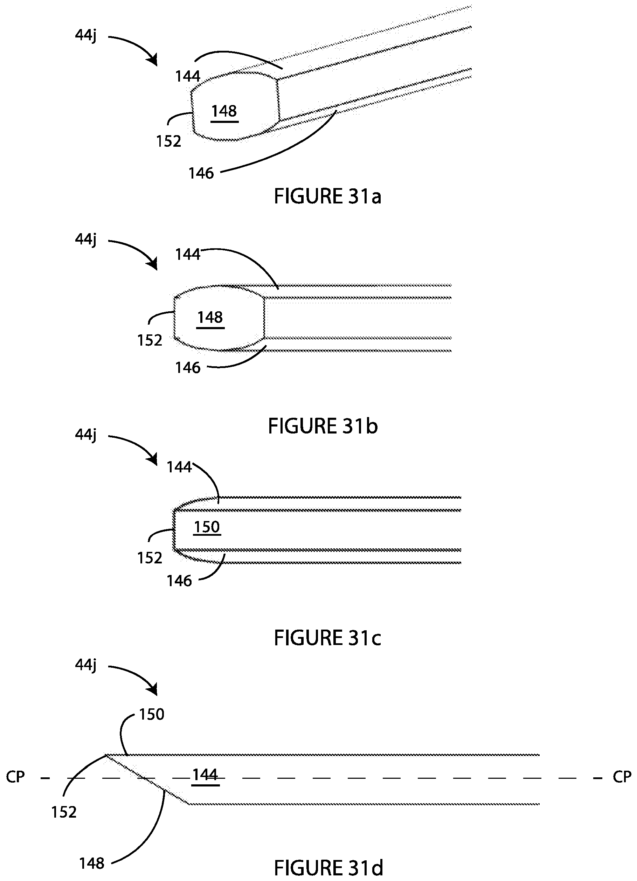

Figures 31a-31d show another alternative design of a stylet head 44j located at the distal stylet end 50 of the stylet 14. The stylet head 44j is similar to the stylet head 44i shown in Figures 30a-30c in that it includes the top curved surface 144, the bottom curved surface 146, the first forging surface 148, and the second forging surface 150. However, unlike stylet head 44i, the first and second forging surfaces 148, 150 are not symmetrical about the central plane CP. Instead, the second forging surface 150 extends substantially straight from one of the flats 54. The cutting edge 152 is offset from the central plane CP. The forging surfaces 148, 150 converge to form a chisel-type blade. The stylet head 44j is designed to cut only soft tissue abutting the cutting edge 152. The radiologist can cut soft tissue using the cutting edge 152 in a number of ways. For example, the radiologist can cut soft tissue using the cutting edge 152 by adjusting the medical instrument 10 from the retracted configuration to the protracted configuration. Alternatively, the radiologist can cut soft tissue using the cutting edge 152 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. Depending on the orientation of the stylet 14 within the hypodermic needle 12, the stylet may need to be rotated about the stylet axis 52. A person of ordinary skill in the art will understand that the foregoing list of examples of how a radiologist can cut soft tissue using stylet head 44j is not exhaustive.

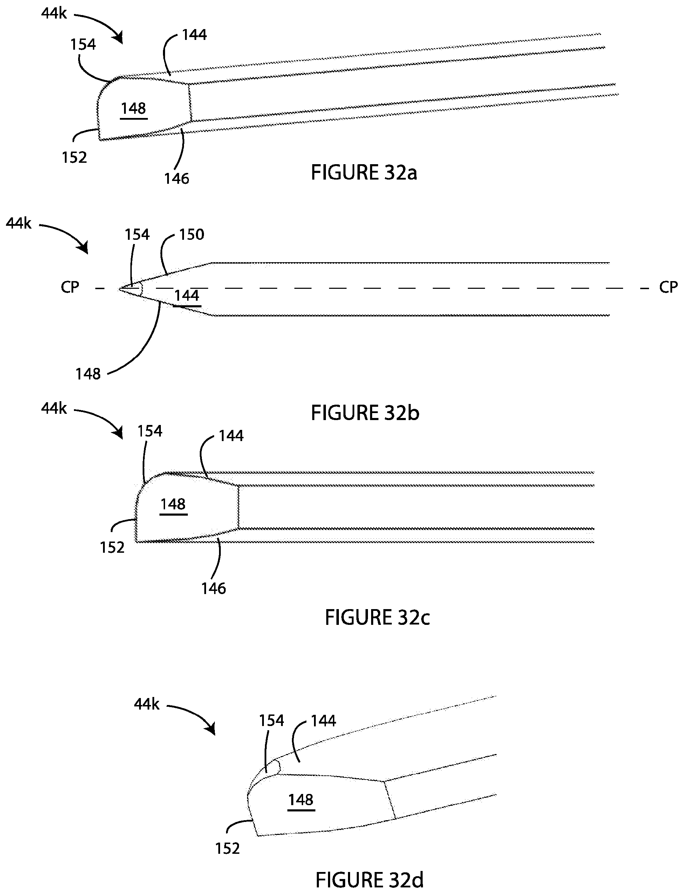

Figures 32a-32d show another alternative design of a stylet head 44k located at the distal stylet end 50 of the stylet 14. The stylet head 44k is similar to the stylet head 44i shown in Figures 30a-30c, with the exception that the intersection between the top curved surface 144 and the first and second forging surfaces 148, 150 forms an atraumatic corner region 154. The atraumatic corner region 154 can move soft tissue without damaging or cutting the soft tissue. The atraumatic corner region 154 is oriented such that the tip is symmetrical about the central plane CP. The stylet head 44i is designed to cut only soft tissue abutting the cutting edge 152. The radiologist can cut soft tissue using the cutting edge 152 in a number of ways. For example, the radiologist can cut soft tissue using the cutting edge 152 by adjusting the medical instrument 10 from the retracted configuration to the protracted configuration. Alternatively, the radiologist can cut soft tissue using the cutting edge 152 by placing and holding the stylet 14 in the protracted configuration and moving the entire medical instrument 10 (including the hypodermic needle 12 and the stylet) in a proximal/distal direction and/or a superficial/deep direction, thereby cutting soft tissue. Depending on the orientation of the stylet 14 within the hypodermic needle 12, the stylet may need to be rotated about the stylet axis 52. A person of ordinary skill in the art will understand that the foregoing list of examples of how a radiologist can cut soft tissue using stylet head 44k is not exhaustive. Additionally, a person of ordinary skill in the art will understand that stylet head 44k could have a second atraumatic tip at the intersection between the bottom curved surface 146 and the first and second forging surfaces 148, 150. Moreover, a person of ordinary skill in the art will understand that stylet head 44j could also have one or two atraumatic tips.

### Interventional Radiology Procedure - Carpal Tunnel Release

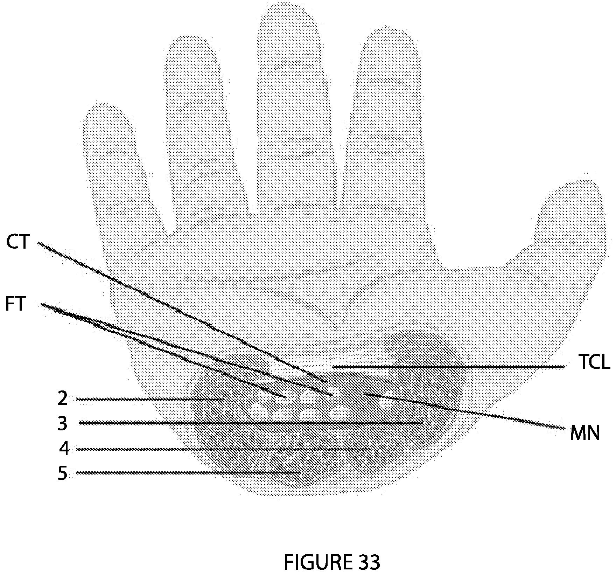

As discussed above, the medical instrument 10 can be used for various reasons within various types of interventional radiology procedures, depending on a number of factors (e.g., size of the hypodermic needle, type of fluid being pushed through the medical instrument, imaging method, etc.). One interventional radiology procedure for which the medical instrument 10 is particularly suited is ultrasound guided carpal tunnel release. The carpal tunnel CT is illustrated in Figure 33. The image shown in Figure 33 is adapted from "Anatomy and Physiology," May 2, 2019 Openstax, available for free download at http://cnx.org/contents/ccc4ed14-6c87-408b-9934-7a0d279d853a@8.

Carpal tunnel syndrome involves compression of a patient's median nerve MN deep in the wrist. Most commonly, the patient's median nerve MN is compressed by the transverse carpal ligament TCL (also referred to as the flexor retinaculum). The TCL attaches to the hook of hamate (labeled as element 2) and the trapezium (labeled as element 3). The TCL forms the roof of the carpal tunnel located on the volar aspect of the wrist. As seen in Figure 33, the median nerve is deep to the TCL. Other anatomical elements are the flexor tendons FT, trapezoid (labeled as element 4), and the capitate (labeled as element 5).

Most often, a patient experiencing carpal tunnel syndrome is prescribed nonsurgical methods in an attempt to remediate the compression of the median nerve. These nonsurgical methods may include rest, splinting, physical therapy, and corticosteroid injections. If one or more of the aforementioned nonsurgical methods fails to remediate the compression of the median nerve MN, a release of the median nerve MN may be achieved by sectioning the TCL. Historically, the TCL has been sectioned using open surgery. Open carpal tunnel releases, however, have a number of drawbacks. For example, open surgery is invasive and requires a large incision (often times more than 60 mm in length). The large incision increases scarring, the risk of infection, and the risk of complication during the surgery. It also increases the recovery period for a patient. Additionally, an open carpal tunnel release must be performed in an operating room and requires multiple specialists to be present (e.g., orthopedic surgeon and an anesthesiologist). The necessity to perform an open carpal tunnel release in an operating room with multiple specialists present dramatically increases medical costs associated with the procedure.

Using the medical instrument 10, a radiologist can perform a minimally invasive carpal tunnel release using an interventional radiology procedure, thereby avoiding the need to perform an open carpal tunnel release. The radiologist maintains direct visualization of the patient's affected wrist throughout the entirety of the carpal tunnel release procedure. Direct visualization enables the radiologist to guide the medical instrument 10 to the appropriate location within the patient without damaging any nerves and/or blood vessels. Although this disclosure describes certain exemplary methods of performing carpal tunnel release as being conducted by a radiologist, it is to be understood that other practitioners or medical health professionals could conduct one or more aspects of any of the methods described herein.

Direct visualization can be achieved through several different types of interventional imaging methods, including, for example, X-ray fluoroscopy, computed tomography (CT), ultrasound, and magnetic resonance imaging (MM). The imaging method discussed throughout the remaining portion of the detailed description will be ultrasound. A person of ordinary skill in the art will understand, however, that other suitable imaging methods could be used in accordance with the method disclosed herein.

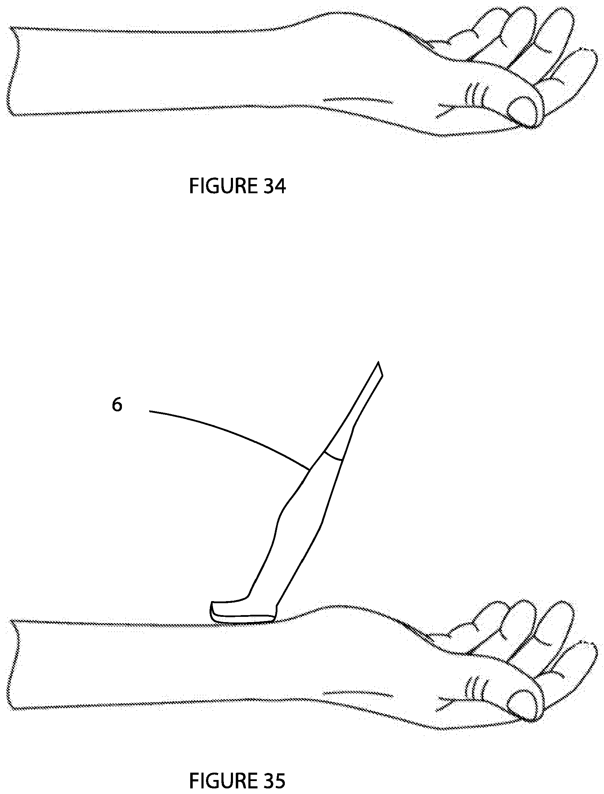

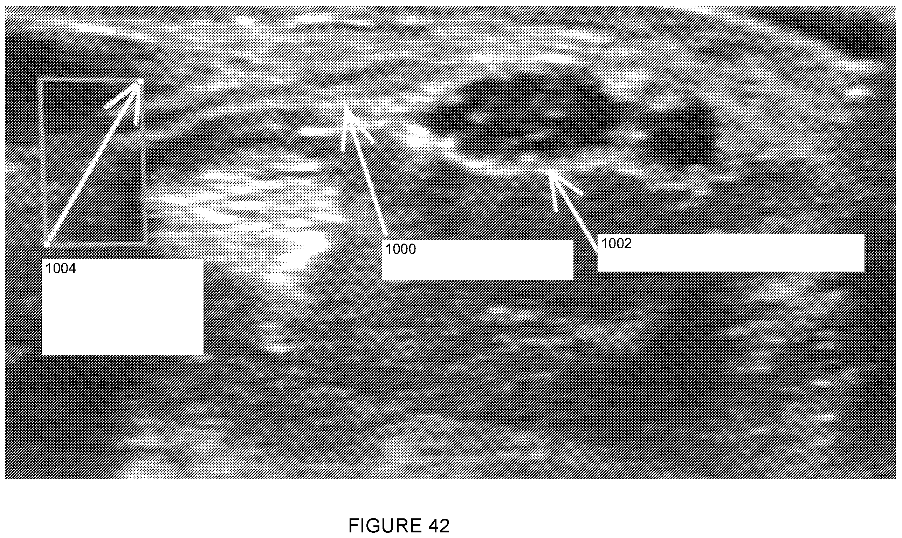

At the beginning of the interventional radiology procedure, a patient experiencing symptoms of carpal tunnel syndrome is placed in a supine position or a recumbent position, depending upon the circumstances. For example, a radiologist may prefer to place the patient in either a supine position or a sitting position depending upon available room equipment (e.g., chair or bed) and room layout. The patient's affected wrist is oriented such that the palm correlating to the affected wrist is facing upwards (i.e., palmar), as illustrated in Figure 34. An ultrasound transducer 6 is placed on a palmar side of the patient's wrist to enable the radiologist to determine the cross-sectional anatomy of the patient's wrist, as illustrated in Figure 35. A person of ordinary skill in the art will understand that the probe may be placed longitudinally on the patient's wrist or transversely, depending upon the imaging desired by the radiologist. Preferably, the ultrasound transducer is a high frequency transducer (e.g., 15-7 MHz transducer). For example, the ultrasound transducer may be a high frequency, small footprint linear array transducer (commonly referred to as a "hockey stick" transducer). One such type of an ultrasound transducer is Philips L15-7io broadband compact linear array transducer. It is to be understood that the radiologist could be holding and maneuvering the ultrasound transducer with one hand throughout the procedure, enabling the radiologist to hold and maneuver the medical instrument 10 in the opposite hand. Alternatively, an assistant (e.g., nurse or radiology technologist) could be handling and maneuvering the ultrasound transducer throughout the procedure. As can be seen in the ultrasound image shown in Figure 42, some of the primary anatomical structures to be viewed by the radiologist are: (i) TCL (labeled as "1000" in the image"); (ii) muscle tendons; and the (iii) median nerve (labeled as "1002"). In the ultrasound image shown in Figure 42, a portion of a hypodermic needle (labeled as "1004") being introduced into the patient can be seen.

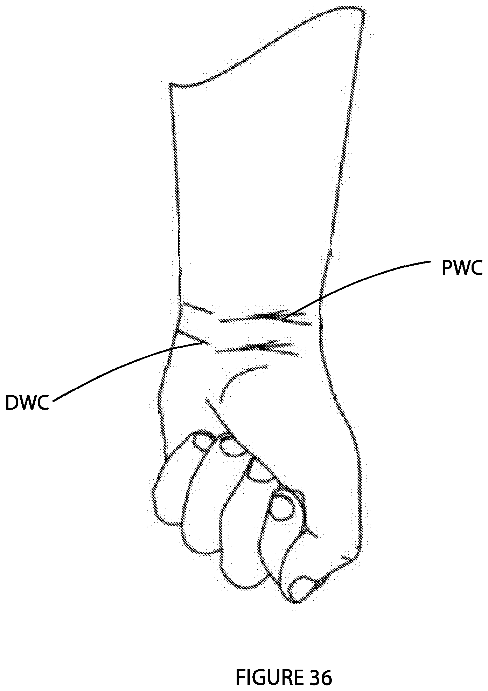

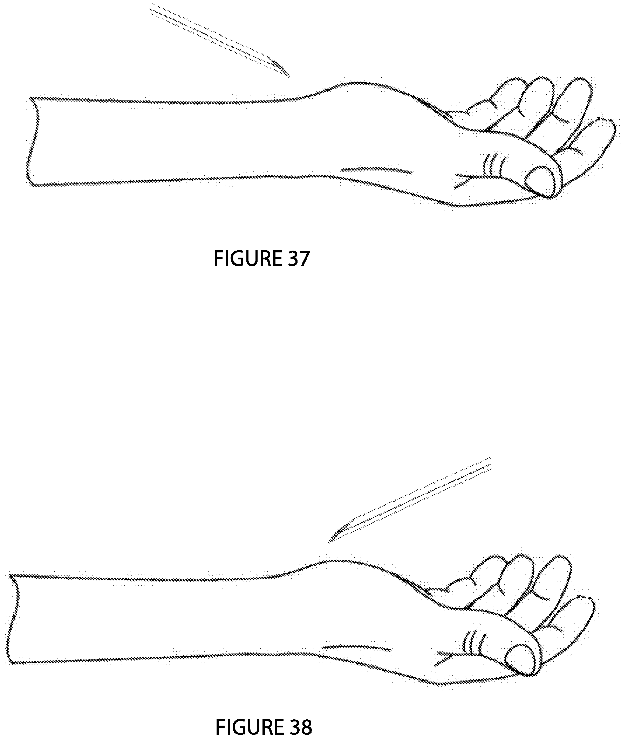

After the radiologist visualizes the anatomical structures of the patient's affected wrist, a first hypodermic needle (referred to from hereon as the "numbing needle") may be introduced through a wrist crease of the affected wrist. The wrist crease can be either the proximal wrist crease PWC or the distal wrist crease DWC, as illustrated in Figure 36. A fluid fitting (e.g., a Luer lock) may be connected to a proximal end of the numbing needle. The fluid fitting enables a syringe containing numbing fluid to be fluidly connected with the numbing needle. A person of ordinary skill in the art will understand that the numbing fluid may contain, for example, a mixture of saline, lidocaine, and/or triamcinolone acetonide. The numbing fluid serves the purpose of numbing the patient's affected anatomy to ensure the patient will remain still during the interventional radiology procedure and to ensure the patient's comfort during the procedure. A person of ordinary skill in the art will understand that the numbing needle may be a small needle because the numbing needle is the first hypodermic needle introduced into the patient. The numbing needle may be introduced at an acute angle to the skin surface. A person of ordinary skill in the art will understand that the numbing needle could be introduced at an angle perpendicular to the skin surface. Under ultrasonographic guidance, the numbing needle is guided to a deep surface of the TCL while ensuring that a distal end of the hypodermic needle does not engage or contact the median nerve. Depending on the circumstances, the radiologist may elect not to pierce the TCL (i.e., remain on the superficial surface of the TCL) with the numbing needle. A person of ordinary skill in the art will understand that the numbing needle may be introduced into the patient proximal-to-distal relative to the affected wrist (shown in Figure 37) or distal-to-proximal relative to the affected wrist (shown in Figure 38). The numbing fluid is at least intermittently injected through the numbing needle as the numbing needle is guided to the TCL.

After the patient is sufficiently anesthetized, the radiologist removes the numbing needle from the patient and introduces the medical instrument 10 into the patient with the stylet 14 in the retracted configuration. A syringe containing fluid is connected to the fluid fitting 18 of the medical instrument 10. A person of ordinary skill in the art will understand that the fluid may be saline because the patient has already been anesthetized. Alternatively, the fluid may be a numbing fluid containing, for example, a mixture of saline, lidocaine, and/or triamcinolone acetonide. The medical instrument 10 is used during the interventional radiology procedure to perform the carpal tunnel release. For this reason, the hypodermic needle 12 associated with the medical instrument 10 will likely be larger in size than the numbing needle. In a preferred embodiment of performing the carpal tunnel release using the method described herein, the numbing needle is a 23-gauge hypodermic needle or greater on the Birmingham gauge. For example, the numbing needle has an outer diameter of less than or equal to about 0.75 mm in one or more embodiments (e.g., less than or equal to about 0.70 mm, less than or equal to 0.65 mm). The hypodermic needle 12 associated with the medical instrument 10 has a gauge number on the Birmingham gauge of less than or equal to 21. For example, the hypodermic needle 12 associated with the medical instrument 10 has an outer diameter of at least about 0.75 mm in one or more embodiments (e.g., at least about 0.80 mm). Using a larger needle for the hypodermic needle 12 enables the radiologist to use a larger, more robust stylet 14 to perform the carpal tunnel release. Suitably, however, the hypodermic needle 12 associated with the medical instrument 10 is also sufficiently small in cross-sectional size to navigate the carpal tunnel anatomy under ultrasound guidance without inadvertently damaging, for example, nerves or blood vessels. In one or more embodiments, the hypodermic needle 12 associated with the medical instrument 10 has an outer diameter of less than or equal to 2.5 mm (e.g., less than or equal to about 2.0 mm, less than or equal to about 1.7 mm, less than or equal to about 1.5 mm, less than or equal to about 1.4 mm). In one or more embodiments, the hypodermic needle 12 associated with the medical instrument 10 comprises one of a 16-guage, 17-gauge, 18-guage, 19-guage, 20-guage, 21-guage, and 22-guage needle on the Birmingham gauge or otherwise comprises a needle of comparable external cross-sectional size to any in this group or any subset of this group of Birmingham needles. A person of ordinary skill in the art will understand that the hypodermic needle 12 associated with the medical instrument 10 could be used to perform the anesthetization in lieu of the numbing needle.

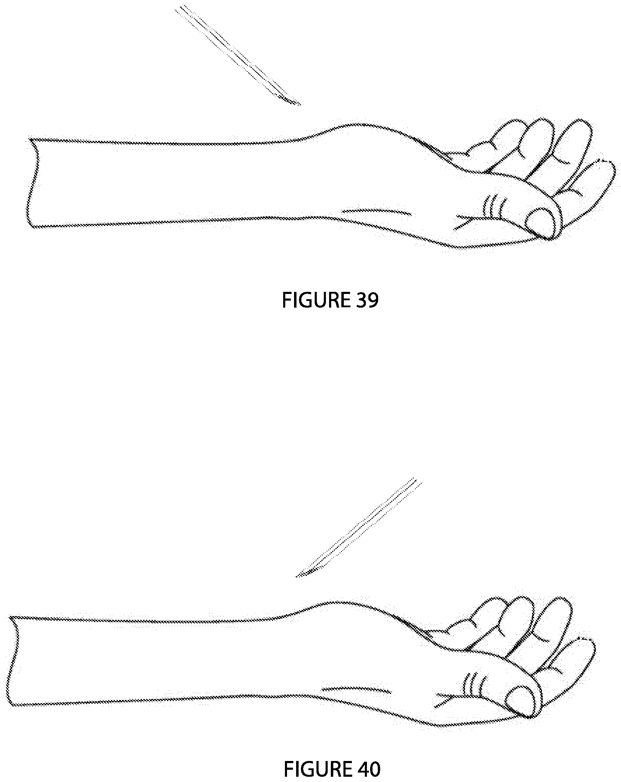

The radiologist may introduce the hypodermic needle 12 associated with the medical instrument 10 through the same entry point used to introduce the numbing needle. As such, the hypodermic needle 12 associated with the medical instrument 10 is introduced through a wrist crease of the affected wrist in one or more embodiments. Similar to the numbing needle, the hypodermic needle 12 associated with the medical instrument 10 can be introduced into the patient generally in the proximal-to-distal direction (see Figure 37) or generally in the distal-to-proximal direction (see Figure 38). The hypodermic needle 12 associated with the medical instrument 10 is introduced such that the sharpened distal tip 40 is the first portion of the hypodermic needle introduced into the patient. As discussed above with regard to the numbing needle, the hypodermic needle 12 associated with the medical instrument 10 may be introduced into the patient at an angle perpendicular to the skin surface. The radiologist, however, will likely introduce the hypodermic needle 12 associated with the medical instrument 10 at an angle acute to the skin surface. The hypodermic needle 12 associated with the medical instrument 10 may be oriented in a bevel up orientation (as shown in Figures 37-38) or a bevel down orientation (as shown in Figures 39-40), depending upon radiologist preference and/or the circumstances associated with the affected wrist. Throughout the interventional radiology procedure, a portion of the hypodermic needle 12 associated with the medical instrument 10 will be positioned at an extracorporeal location.

Under continuous ultrasonographic guidance, the hypodermic needle 12 associated with the medical instrument 10 is guided along the anesthetized track until the sharpened distal tip 40 is immediately superficial of the TCL. In an embodiment, as the radiologist is advancing the hypodermic needle 12 associated with the medical instrument 10 along the anesthetized track, fluid is at least intermittently injected through the needle bore 38. Intermittently injecting fluid helps the radiologist better identify the exact positioning of the hypodermic needle 12 associated with the medical instrument 10 relative to various anatomic structures within the patient's body. Depending upon the circumstances, the fluid could be, for example, saline. Alternatively, the fluid may be a numbing fluid containing, for example, a mixture of saline, lidocaine, and triamcinolone acetonide. Intermittently injecting fluid containing a local anesthetic provides the additional benefit of ensuring the patient remains numb throughout the procedure.

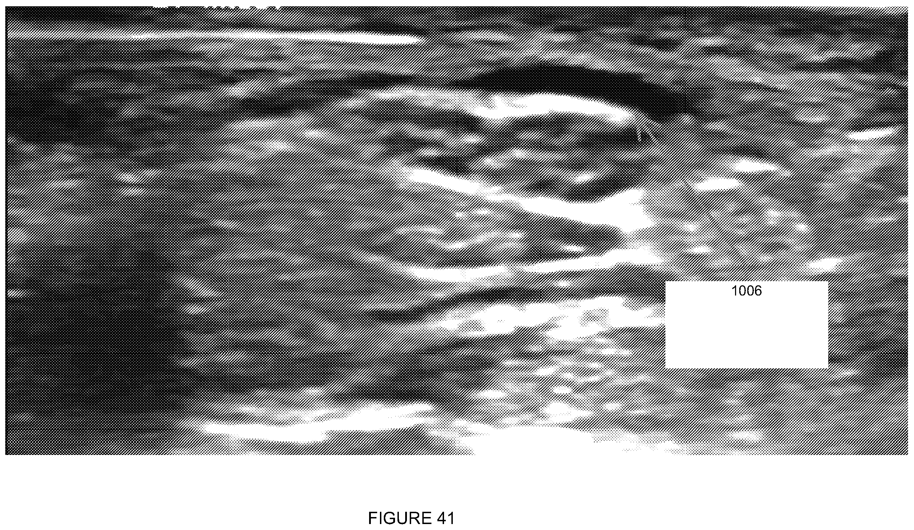

After positioning the hypodermic needle 12 such that the sharpened distal tip 40 is immediately superficial of the TCL, the radiologist subsequently advances the hypodermic needle in a deep direction while injecting fluid through the needle bore 38. This results in hydrodissection as the sharpened distal tip 40 pierces the TCL. The jet of fluid expelled from the hypodermic needle 12 separates the median nerve from the deep surface of the TCL. Continued injection of fluid after piercing the TCL forcibly pushes the median nerve away from the TCL (e.g., by the pressure of the injected fluid acting against the median nerve) and provides a fluid pocket 1006 that isolates the median nerve, as shown in Figure 41. The fluid pocket provides the radiologist enough space to release the TCL using the medical instrument 10 without contacting and/or damaging the median nerve. In one or more embodiments, the fluid pocket is free of solid blocking structure (e.g., structure other than the distal end portion of the shaft of the needle) between the TCL and the median nerve. For example, no solid blocking structure is intentionally introduced to the fluid pocket to form a guard between the needle and the median nerve in certain embodiments. Rather, in these embodiments, the fluid pocket is used to provide ample clearance for the radiologist to conduct a TCL dissection under ultrasound guidance without substantial risk of damaging the median nerve. The fluid pocket can be maintained throughout the remaining aspects of the interventional radiology procedure by injecting additional fluid through the needle bore 38, as necessary. In one or more embodiments, the fluid pocket defines a gap between the median nerve and the TCL of on the order of 1.0 mm to 2.0 mm.

The radiologist subsequently adjusts the medical instrument 10 from the retracted configuration to the protracted configuration such that the stylet head 44 is at least partially positioned within the fluid pocket. A person of ordinary skill in the art will understand that the radiologist may move the hypodermic needle 12 superficial after creating the fluid pocket but before adjusting the stylet 14 from the retracted configuration to the protracted configuration. Alternatively, a person of ordinary skill in the art will understand that the radiologist may adjust the stylet 14 from the retracted configuration to the protracted configuration without moving the hypodermic needle 12 superficially. It should be further understood that, depending on the circumstances, various types of stylet head designs may be used to perform the carpal tunnel release procedure. One type of stylet head design that can be used for cutting the TCL and releasing the median nerve is stylet head 44a shown in Figures 22a-22c.

Depending on the orientation of the stylet head 44a relative to the hypodermic needle 12, the stylet may need to be rotated about the stylet axis 52 to position the cutting edge 104 adjacent the TCL as the medical instrument 10 is adjusted from the retracted configuration to the protracted configuration. For example, the stylet 14 may be oriented within the hypodermic needle 12 such that as the medical instrument 10 is adjusted from the retracted configuration to the protracted configuration, the cutting edge 112 is not adjacent the TCL. Orienting the stylet 14 in this manner may help the radiologist ensure the TCL is not cut by the cutting edge 112 as the medical instrument 10 is adjusted from the retracted configuration to the protracted configuration. After the medical instrument 10 is adjusted to the protracted configuration, the radiologist may rotate the stylet 14 about the stylet axis 52 to position the cutting edge 112 immediately adjacent the TCL. The radiologist may then place the cutting edge 112 into contact with the TCL and move the cutting edge relative to the TCL, thereby cutting the TCL. It is to be understood the cutting edge 112 may be moved in a reciprocating motion while urging the cutting edge against the TCL. The reciprocating motion causes the cutting edge 112 of the stylet head 44a to wear against the TCL until the TCL is dissected. Alternatively, the radiologist may cut the TCL with the cutting edge 104 by adjusting the stylet 14 from the protracted configuration to the retracted configuration. Using the medical instrument 10 in this manner ensures that the stylet 14 is sheathed or housed within the hypodermic needle 12 after cutting the TCL and releasing the median nerve. Subsequent to releasing the median nerve and the stylet head 44a being housed within the hypodermic needle 10 (i.e., moved to the retracted position), the radiologist may withdraw the medical instrument 10 from the patient. Because the stylet head 44a is in the retracted position, the patient is protected when withdrawing the medical instrument 10.

Alternatively, the stylet head 44a may be oriented within the hypodermic needle 12 such that as the stylet 14 is adjusted from the retracted configuration to the protracted configuration, the cutting edge 112 is adjacent to, and in contact with, the TCL. Having the stylet 14 oriented in this manner may enable the radiologist to make a first cutting pass on the TCL as the medical instrument 10 is adjusted from the retracted configuration to the protracted configuration. The radiologist can then make a second cutting pass on the TCL using the cutting edge 112 as the medical instrument 10 is moved from the protracted configuration to the retracted configuration. The two passes may help ensure the TCL is fully dissected and the median nerve is released, while also ensuring the stylet head 44a is sheathed or housed within the hypodermic needle 12 after cutting the TCL and releasing the median nerve.

While dissecting the TCL, the radiologist may intermittently inject fluid through the medical instrument 10. Injection of fluid assists with the dissection because the jet of fluid expelled from the distal needle end 34 of the hypodermic needle 12 helps force severance of the TCL. In many instances, the TCL has thickened such that it is taut about the median nerve. Thus, the combination of the cutting edge 112 of the stylet 14 repeatedly wearing against the TCL and the jet of fluid expelled from the distal needle end 34 will provide enough force to sever the taut TCL and release the median nerve. Injection of fluid while dissecting the TCL also enables the radiologist to identify when the TCL has been dissected. After the TCL has been severed, fluid being expelled from the hypodermic needle 12 will cause the TCL to flutter. This fluttering of the TCL provides the radiologist visual indication via ultrasonic guidance that the TCL has been cut and the median nerve released.

The radiologist may then remove the syringe connected with the fluid fitting 18 and replace the syringe with a second syringe containing a steroid fluid. The steroid fluid could be, for example, a corticosteroid such as triamcinolone acetonide. The second syringe is connected to the fluid fitting 18 such that the fluid fitting (and therefore the hypodermic needle 12) is fluidly connected with the steroid fluid. The radiologist may then inject the steroid fluid into the patient at the localized area where the TCL was dissected. Because the affected wrist was not "opened" as is the case in open surgery, the steroid fluid can be readily absorbed by the soft tissue of the patient. Injection of the steroid fluid helps prevent or lessen the inflammatory response of the patient as a result of the dissected TCL. This hinders the potential development of post procedural fibrosis or scar formation. Directing the steroid fluid through the hypodermic needle 12 associated with the medical instrument 10, rather than a new hypodermic needle inserted into the patient, ensures the steroid fluid is directed to the localized area where the TCL was dissected. In some instances, scarring of the TCL can result in the reoccurrence of carpal tunnel syndrome. The radiologist may then remove the second syringe from the fluid fitting and replace the second syringe with another syringe containing a non-steroid fluid (e.g., flushing fluid such as lidocaine or saline). This syringe, which is fluidly connected with the fluid fitting, enables the radiologist to flush the procedure needle of any steroid fluid before bringing the hypodermic needle 12 superficially to the patient's skin. Bringing steroid fluid superficially to the patient's skin can, in some instances, result in skin irritation. After flushing the hypodermic needle 12, the radiologist may remove the medical instrument 10 from the patient and place a small bandage at the point of entry, if necessary.

Using the medical instrument 10 and the interventional radiology procedure described above to dissect the TCL provides for a minimally invasive carpal tunnel release. While multiple entry points may be used throughout the entirety of the procedure (e.g., numbing needle may have a different entry point than the hypodermic needle 12 associated with the medical instrument 10), access for dissecting the TCL (or for both moving the median nerve and dissecting the TCL) can be provided through one, and only one, entry point in the hand/wrist of the patient. Unlike open carpal tunnel release in which the entry point is an incision that is in some instances multiple centimeters or inches in length, or even certain less invasive carpal tunnel release procedures that use smaller incisions on the order of 4 mm or greater, the entry point for dissecting the TCL in the procedure described herein is only a hypodermic needle puncture. Thus, in one or more embodiments, the entry point (e.g., hypodermic needle puncture) for an instrument which dissects a TCL has a maximum transverse dimension of less than 3.5 mm, less than 3.0 mm, less than 2.5 mm, less than 2.0 mm, less than 1.7 mm, less than 1.5 mm, or less than 1.4 mm. The small transverse dimension of the entry point facilitates conducting a carpal tunnel release procedure in a minimally invasive manner. The minimally invasive nature helps reduce the risk of infection, enables the procedure to be performed during an office visit at an outpatient facility (which helps reduce medical costs), drastically minimizes recovery time necessary for the entry point to heal, and significantly reduces the risk of scarring (both on the skin surface and internally at the location where the TCL is dissected).

It is to be understood that other types of stylet head designs that can be used for performing a carpal tunnel release other than stylet head 44a. Depending on the stylet head 44 and the positioning of the cutting edge on said stylet head, a person of ordinary skill in the art will understand that the exact procedure for dissecting the TCL may differ from that provided above. For example, if stylet head 44c is being used to dissect the TCL, the stylet 14 could be used in a manner such that the TCL is positioned within the hook region 118, thereby enabling cutting edge 124 to dissect the TCL.

### Other Interventional Radiology Procedures

It will be apparent to a person skilled in the art that the medical instrument 10 is suitable for use in other types of image-guided radiology procedures that involve manipulating soft tissue. In general, during any image-guided radiology procedure, the radiologist at least intermittently views the target anatomy with a form of imaging such as ultrasound, Mill, or the like. In some embodiments, the radiologists uses a numbing needle to establish an anesthetized track before introducing the medical instrument 10. To introduce the medical instrument, the sharpened tip of the hypodermic needle 12 pierces the skin of the patient or otherwise enters the body of the patient through an appropriate entry point. Then the needle 12 is advanced under image guidance until the needle distal end is located at the target site. At any time while advancing the needle 12, fluid can be continuously or intermittently imparted through the needle along the stylet 14 such that is discharged from needle distal end to achieve any desired effect, e.g., hydro-dissection, therapeutic treatment of tissue, anesthetization, image enhancement or improved visualization. When imaging shows the needle distal end to be at the target site, a stylet 14 with the desired stylet head is advanced through the needle bore to the protracted configuration. Subsequently, the medical instrument 10 is moved as a unit or the stylet 14 is moved relative to the needle 12 to manipulate the target tissue under image guidance as required in the procedure. At any time while using the stylet head to manipulate tissue, fluid can be continuously or intermittently imparted through the needle along the stylet 14 such that is discharged from needle distal end to achieve a desired effect, e.g., hydro-dissection, therapeutic treatment of tissue, anesthetization, image enhancement or improved visualization. Syringes containing any desired fluid can be coupled to the fluid fitting 18 and imparted through the needle 12 during the procedure. When the procedure is complete, the needle can be withdrawn from the patient.

Because a needle puncture is the sole entry point used for the procedure, suturing is typically not required and patient recovery can involve minimal pain and discomfort. Unlike open surgery, including less invasive surgical procedures that use smaller incisions on the order of 4 mm or greater, the entry point used to conduct procedures with the medical instrument 10 is only a hypodermic needle puncture. Thus, in one or more embodiments, the entry point (e.g., hypodermic needle puncture) for conducting a interventional radiology procedure using the medical instrument 10 has a maximum transverse dimension of less than 3.5 mm, less than 3.0 mm, less than 2.5 mm, less than 2.0 mm, less than 1.7 mm, less than 1.5 mm, or less than 1.4 mm. The small transverse dimension of the entry point facilitates conducting the procedure in a minimally invasive manner. The minimally invasive nature helps reduce the risk of infection, enables the procedure to be performed during an office visit at an outpatient facility (which helps reduce medical costs), drastically minimizes recovery time necessary for the entry point to heal, and significantly reduces the risk of scarring (both on the skin surface and internally at the location where the TCL is dissected).

Among other interventional radiology procedures that can be performed using the medical instrument 10, it is expressly contemplated that the instrument is used in the general manner described above to conduct procedures comprising De Quervain release, trigger finger release, tarsal tunnel release, plantar fascia release, arm or leg fasciotomy, a lavage (e.g., shoulder lavage), and tissue biopsy (e.g., pancreatic biopsy). In view of the foregoing, basic methods of using the medical instrument 10 to conduct these and other procedures will be apparent to a person skilled in the art.

For example, to conduct a De Quervain release, the hypodermic needle 12 is introduced under image guidance into the hand or wrist toward the affected tendons running alongside the wrist near the thumb. When the distal end of the needle is at the target site, the stylet having the desired stylet head configuration is advanced to the protracted configuration and used to release the affected tendons (with or without the aid of hydro-dissection or other therapeutic or image-enhancing fluids imparted through the needle bore during the procedure).

To conduct a trigger finger release, the hypodermic needle 12 is introduced under image guidance into the hand toward the annular ligament. When the distal end of the needle is at the target site (e.g., deep of the annular ligament), the stylet having the desired stylet head configuration is advanced to the protracted configuration and used to release the affected tendons (with or without the aid of hydro-dissection or other therapeutic or image-enhancing fluids imparted through the needle bore during the procedure).

To conduct a tarsal tunnel release, the hypodermic needle 12 is introduced under image guidance into the foot or ankle toward the tarsal ligament. When the distal end of the needle is at the target site, the stylet having the desired stylet head configuration is advanced to the protracted configuration and used to release the affected tendons (with or without the aid of hydro-dissection or other therapeutic or image-enhancing fluids imparted through the needle bore during the procedure).

To conduct a plantar fasciitis release, the hypodermic needle 12 is introduced under image guidance into the foot or ankle toward the plantar fascia. When the distal end of the needle is at the target site, the stylet having the desired stylet head configuration is advanced to the protracted configuration and used to release the plantar fascia (with or without the aid of hydro-dissection or other therapeutic or image-enhancing fluids imparted through the needle bore during the procedure).

To conduct an arm or leg fasciotomy, the hypodermic needle 12 is introduced under image guidance into the arm or leg toward the respective fascia at a plurality of locations longitudinally spaced apart along the arm or leg. When the distal end of the needle is at each target site, the stylet having the desired stylet head configuration is advanced to the protracted configuration and used to release the fascia (with or without the aid of hydro-dissection or other therapeutic or image-enhancing fluids imparted through the needle bore during the procedure).

To conduct a shoulder lavage, the hypodermic needle 12 is introduced under image guidance into the arm or shoulder. (It will be appreciated that the needle is introduced into other body parts when other lavages are to be conducted). Using the medical instrument, the shoulder lavage can be conducted generally using conventional lavage techniques except that the stylet head is used to break calcific deposits in the shoulder. During the procedure flushing fluid (e.g., saline) passed through the needle along the stylet is used to flush the calcium from the joint area. Subsequently, a steroid is passed through the needle along stylet into the joint area (e.g., into the bursa).

During a biopsy, the hypodermic needle 12 is introduced under image guidance toward the part of the anatomy to be biopsied. When the distal end of the needle is at the target site, the stylet having the desired stylet head configuration is advanced to the protracted configuration and used to extract samples of the target tissue. In one or more embodiments, a tissue sample is retained on the stylet head, which is then retracted. The collected tissue sample is then safely sheathed within the needle until the medical instrument is withdrawn.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above products and methods without departing from the scope of the invention, it is intended that all matter contained in the above description shall be interpreted as illustrative and not in a limiting sense.

## Claims

1. A medical instrument (10) for cutting soft tissue during an interventional radiology procedure, the medical instrument (10) comprising:
a hypodermic needle (12) having an inner needle surface (28), an outer needle surface (30), a proximal needle end (32), a distal needle end (34), and a needle axis (36), the inner needle surface (28) defining a needle bore (38) extending from the proximal needle end (32) to the distal needle end (34), the distal needle end (34) having a sharpened distal tip (40) configured to puncture soft tissue;
a stylet (14) having a stylet body (42), a stylet head (44), an outer stylet surface (46), a proximal stylet end (48), a distal stylet end (50), and a stylet axis (52), the stylet axis (52) being coaxial with the needle axis (36), the stylet head (44) being located at the distal stylet end (50), at least a portion of the stylet (14) being located within the needle bore (38), the portion of the stylet (14) located within the needle bore (38) and the inner needle surface (28) of the hypodermic needle (12) collectively forming at least one fluid passageway (56), the stylet (14) being movable relative to the hypodermic needle (12) along the needle axis (36), the stylet (14) being adjustable between a retracted configuration and a protracted configuration, the stylet head (44) being located within the needle bore (38) when the medical instrument (10) is in the retracted configuration, the stylet head (44) being located external to the needle bore (38) when the medical instrument (10) is in the protracted configuration, the fluid passageway (56) enabling fluid to flow from the proximal needle end (32) to the distal needle end (34) when the stylet (14) is in the retracted configuration and in the protracted configuration, the fluid passageway (56) being configured such that fluid in the fluid passageway (56) flows between the outer stylet surface (46) and the inner needle surface (28);
wherein the sharpened distal tip (40) enables the hypodermic needle (12) to easily puncture the skin of a patient to gain access to the human anatomy; and
the medical instrument (10) is configured such that unlike open surgery, including less invasive surgical procedures that use smaller incisions on the order of 4 mm or greater, an entry point used to conduct the procedure with the medical instrument (10) is only a hypodermic needle puncture having a maximum transverse dimension of less than 3.5 mm.

2. The medical instrument (10) as set forth in claim 1, wherein the hypodermic needle (12) is configured to conform to a size defined by the Birmingham gauge, and wherein the hypodermic needle (12) is smaller than a 14 gauge needle as defined by the Birmingham gauge and larger than a 28 gauge needle as defined by the Birmingham gauge.

3. The medical instrument (10) as set forth in claim 2, wherein the hypodermic needle (12) is smaller than a 17 gauge needle as defined by the Birmingham gauge and larger than a 23 gauge needle as defined by the Birmingham gauge.

4. The medical instrument (10) as set forth in claim 1, wherein the proximal stylet end (48) extends in a proximal direction from the proximal needle end (32), and wherein the medical instrument (10) further comprises a fluid fitting (18) configured to be removably connected to a syringe (86), the fluid fitting (18) being connected to the proximal stylet end (48) of the stylet (14) wherein during use of the medical instrument (10), fluid can be directed from the syringe (86) through the needle bore (38) and along the stylet (14) so that the fluid can be discharged from the distal needle end (34).

5. The medical instrument (10) as set forth in claim 4, wherein the medical instrument (10) further comprises a collar (16), the collar (16) being fixedly connected to the proximal needle end (32).

6. The medical instrument (10) as set forth in claim 5, wherein the collar (16) encircles at least a portion of the stylet (14), the stylet (14) and the fluid fitting (18) being movable relative to the collar (16).

7. The medical instrument (10) as set forth in claim 6, wherein the collar (16) is configured to accommodate at least a portion of the fluid fitting (18).

8. The medical instrument (10) as set forth in claim 7, wherein the medical instrument (10) further comprises a collar assembly, the collar assembly including the collar (16) and at least one 0-ring, the 0-ring being positioned within the collar (16).

9. The medical instrument (10) as set forth in claim 8, wherein the collar (16) is fluidly connected to the fluid fitting (18).

10. The medical instrument (10) as set forth in claim 1, wherein the medical instrument (10) is adjustable from a locked configuration to an unlocked configuration, the stylet (14) being fixed relative to the hypodermic needle (12) when in the locked configuration and movable relative to the hypodermic needle (12) when in the unlocked configuration.

11. The medical instrument (10) as set forth in claim 10, wherein the medical instrument (10) is configured such that fluid can flow through the fluid passageway (56) when the medical instrument (10) is in the locked configuration and in the unlocked configuration.

12. The medical instrument (10) as set forth in claim 11, wherein the medical instrument (10) is configured such that the stylet (14) is in the retracted configuration when the medical instrument (10) is in the locked configuration.

13. The medical instrument (10) as set forth in claim 12, wherein the stylet (14) is rotatable about the stylet axis (52).

14. The medical instrument (10) as set forth in claim 13, wherein the medical instrument (10) is configured to adjust from the locked configuration to the unlocked configuration by rotating the stylet (14) about the stylet axis (52).

15. The medical instrument (10) as set forth in claim 1, wherein the stylet head (44) is configured to cut a transverse carpal ligament, and optionally: wherein the stylet (14) is a solid monolithic component devoid of any bores and wherein either: the outer stylet surface (46) of the stylet body (42) includes at least one flat extending through the needle bore (38), the flat of the stylet (14) and the inner needle surface (28) of the hypodermic needle (12) collectively forming the fluid passageway (56); or the outer stylet surface (46) of the stylet body (42) includes at least one notched region extending through the needle bore (38), the notched region of the stylet (14) and the inner needle surface (28) of the hypodermic needle (12) collectively forming the fluid passageway (56).

## Patentansprüche

1. Medizinisches Instrument (10) zum Schneiden von Weichgewebe während eines interventionellen radiologischen Eingriffs, wobei das medizinische Instrument (10) umfasst:
eine Injektionsnadel (12), aufweisend eine innere Nadelfläche (28), eine äußere Nadelfläche (30), ein proximales Nadelende (32), ein distales Nadelende (34) und eine Nadelachse (36), wobei die innere Nadelfläche (28) eine Nadelbohrung (38) definiert, die sich von dem proximalen Nadelende (32) zu dem distalen Nadelende (34) erstreckt, wobei das distale Nadelende (34) eine angeschliffene distale Spitze (40) aufweist, die konfiguriert ist, um Weichgewebe zu punktieren;
ein Stilett (14), aufweisend einen Stilettkörper (42), einen Stilettkopf (44), eine äußere Stilettfläche (46), ein proximales Stilettende (48), ein distales Stilettende (50) und eine Stilettachse (52), wobei die Stilettachse (52) koaxial mit der Nadelachse (36) ist, wobei sich der Stilettkopf (44) an dem distalen Stilettende (50) befindet, wobei sich mindestens ein Abschnitt des Stiletts (14) innerhalb der Nadelbohrung (38) befindet, wobei der Abschnitt des Stiletts (14), der sich innerhalb der Nadelbohrung (38) befindet, und die innere Nadelfläche (28) der Injektionsnadel (12) gemeinsam mindestens einen Fluiddurchlass (56) bilden, wobei das Stilett (14) in Bezug auf die Injektionsnadel (12) entlang der Nadelachse (36) bewegbar ist, wobei das Stilett (14) zwischen einer eingezogenen Konfiguration und einer ausgezogenen Konfiguration justierbar ist, wobei sich der Stilettkopf (44) innerhalb der Nadelbohrung (38) befindet, wenn das medizinische Instrument (10) in der eingezogenen Konfiguration ist, wobei sich der Stilettkopf (44) extern zu der Nadelbohrung (38) befindet, wenn das medizinische Instrument (10) in der ausgezogenen Konfiguration ist, wobei der Fluiddurchlass (56) ermöglicht, dass Fluid von dem proximalen Nadelende (32) zu dem distalen Nadelende (34) strömt, wenn das Stilett (14) in der eingezogenen Konfiguration und in der ausgezogenen Konfiguration ist, wobei der Fluiddurchlass (56) konfiguriert ist, so dass Fluid in dem Fluiddurchlass (56) zwischen der äußeren Stilettfläche (46) und der inneren Nadelfläche (28) strömt;
wobei die angeschliffene distale Spitze (40) ermöglicht, dass die Injektionsnadel (12) die Haut eines Patienten einfach punktiert, um Zugang zu der menschlichen Anatomie zu erlangen; und
das medizinische Instrument (10) konfiguriert ist, so dass im Gegensatz zu offener Chirurgie, einschließlich weniger invasiver chirurgischer Eingriffe, die kleinere Inzisionen in der Größenordnung von 4 mm oder mehr verwenden, ein Eintrittspunkt, der zum Vornehmen des Eingriffs mit dem medizinischen Instrument (10) verwendet wird, nur eine Injektionsnadelpunktion ist, die eine maximale Querabmessung von weniger als 3,5 mm aufweist.

2. Medizinisches Instrument (10) nach Anspruch 1, wobei die Injektionsnadel (12) konfiguriert ist, um einer Größe zu entsprechen, die durch die Birmingham Gauge definiert ist, und wobei die Injektionsnadel (12) kleiner als eine 14-Gauge-Nadel ist, wie durch die Birmingham Gauge definiert, und größer als eine 28-Gauge-Nadel ist, wie durch die Birmingham Gauge definiert.

3. Medizinisches Instrument (10) nach Anspruch 2, wobei die Injektionsnadel (12) kleiner als eine 17-Gauge-Nadel ist, wie durch die Birmingham Gauge definiert, und größer als eine 23-Gauge-Nadel ist, wie durch die Birmingham Gauge definiert.

4. Medizinisches Instrument (10) nach Anspruch 1, wobei sich das proximale Stilettende (48) in einer proximalen Richtung von dem proximalen Nadelende (32) erstreckt, und wobei das medizinische Instrument (10) ferner ein Fluidanschlussstück (18) umfasst, das konfiguriert ist, um abnehmbar mit einer Spritze (86) verbunden zu sein, wobei das Fluidanschlussstück (18) mit dem proximalen Stilettende (48) des Stiletts (14) verbunden ist, wobei Fluid im Gebrauch des medizinischen Instruments (10) aus der Spritze (86) durch die Nadelbohrung (38) und entlang des Stiletts (14) geleitet werden kann, so dass das Fluid aus dem distalen Nadelende (34) abgeführt werden kann.

5. Medizinisches Instrument (10) nach Anspruch 4, wobei das medizinische Instrument (10) ferner einen Kragen (16) umfasst, wobei der Kragen (16) fest mit dem proximalen Nadelende (32) verbunden ist.

6. Medizinisches Instrument (10) nach Anspruch 5, wobei der Kragen (16) mindestens einen Abschnitt des Stiletts (14) umgibt, wobei das Stilett (14) und das Fluidanschlussstück (18) in Bezug auf den Kragen (16) bewegbar sind.

7. Medizinisches Instrument (10) nach Anspruch 6, wobei der Kragen (16) konfiguriert ist, um mindestens einen Abschnitt des Fluidanschlussstücks (18) unterzubringen.

8. Medizinisches Instrument (10) nach Anspruch 7, wobei das medizinische Instrument (10) ferner eine Kragenanordnung umfasst, wobei die Kragenanordnung den Kragen (16) und mindestens einen Dichtungsring umfasst, wobei der Dichtungsring innerhalb des Kragens (16) positioniert ist.

9. Medizinisches Instrument (10) nach Anspruch 8, wobei der Kragen (16) mit dem Fluidanschlussstück (18) fluidverbunden ist.

10. Medizinisches Instrument (10) nach Anspruch 1, wobei das medizinische Instrument (10) aus einer gesperrten Konfiguration in eine entsperrte Konfiguration justierbar ist, wobei das Stilett (14) in Bezug auf die Injektionsnadel (12) fixiert ist, wenn es in der gesperrten Konfiguration ist, und in Bezug auf die Injektionsnadel (12) bewegbar ist, wenn es in der entsperrten Konfiguration ist.

11. Medizinisches Instrument (10) nach Anspruch 10, wobei das medizinische Instrument (10) konfiguriert ist, so dass Fluid durch den Fluiddurchlass (56) strömen kann, wenn das medizinische Instrument (10) in der gesperrten Konfiguration und in der entsperrten Konfiguration ist.

12. Medizinisches Instrument (10) nach Anspruch 11, wobei das medizinische Instrument (10) konfiguriert ist, so dass das Stilett (14) in der eingezogenen Konfiguration ist, wenn das medizinische Instrument (10) in der gesperrten Konfiguration ist.

13. Medizinisches Instrument (10) nach Anspruch 12, wobei das Stilett (14) um die Stilettachse (52) drehbar ist.

14. Medizinisches Instrument (10) nach Anspruch 13, wobei das medizinische Instrument (10) konfiguriert ist, um sich durch Drehen des Stiletts (14) um die Stilettachse (52) aus der gesperrten Konfiguration in die entsperrte Konfiguration zu justieren.

15. Medizinisches Instrument (10) nach Anspruch 1, wobei der Stilettkopf (44) konfiguriert ist, um ein transversales Karpalband zu schneiden, und optional: wobei das Stilett (14) eine massive einteilige Komponente ist, die frei von jeglichen Bohrungen ist, und wobei entweder: die äußere Stilettfläche (46) des Stilettkörpers (42) mindestens eine Abflachung einschließt, die sich durch die Nadelbohrung (38) erstreckt, wobei die Abflachung des Stiletts (14) und die innere Nadelfläche (28) der Injektionsnadel (12) gemeinsam den Fluiddurchlass (56) bilden; oder die äußere Stilettfläche (46) des Stilettkörpers (42) mindestens eine eingekerbte Region einschließt, die sich durch die Nadelbohrung (38) erstreckt, wobei die eingekerbte Region des Stiletts (14) und die innere Nadelfläche (28) der Injektionsnadel (12) gemeinsam den Fluiddurchlass (56) bilden.

## Revendications

1. Instrument médical (10) pour couper des tissus mous lors d'une procédure de radiologie interventionnelle, l'instrument médical (10) comprenant:
une aiguille hypodermique (12) comportant une surface intérieure d'aiguille (28), une surface extérieure d'aiguille (30), une extrémité proximale d'aiguille (32), une extrémité distale d'aiguille (34), et un axe d'aiguille (36), la surface intérieure de l'aiguille (28) définissant un alésage d'aiguille (38) s'étendant de l'extrémité proximale de l'aiguille (32) à l'extrémité distale de l'aiguille (34), l'extrémité distale de l'aiguille (34) comportant une pointe distale aiguisée (40) configurée pour perforer les tissus mous;
un stylet (14) comportant un corps de stylet (42), une tête de stylet (44), une surface extérieure de stylet (46), une extrémité proximale de stylet (48), une extrémité distale de stylet (50), et un axe de stylet (52), l'axe du stylet (52) étant coaxial à l'axe de l'aiguille (36), la tête du stylet (44) étant située à l'extrémité distale du stylet (50), au moins une partie du stylet (14) étant située à l'intérieur de l'alésage de l'aiguille (38), la partie du stylet (14) située à l'intérieur de l'alésage de l'aiguille (38) et la surface intérieure (28) de l'aiguille hypodermique (12) formant ensemble au moins un passage pour fluide (56), le stylet (14) étant mobile par rapport à l'aiguille hypodermique (12) le long de l'axe de l'aiguille (36), le stylet (14) étant réglable entre une configuration rétractée et une configuration déployée, la tête du stylet (44) étant située à l'intérieur de l'alésage de l'aiguille (38) lorsque l'instrument médical (10) est dans la configuration rétractée, la tête du stylet (44) étant située à l'extérieur de l'alésage de l'aiguille (38) lorsque l'instrument médical (10) est dans la configuration déployée, le passage pour fluide (56) permettant au fluide de s'écouler de l'extrémité proximale de l'aiguille (32) à l'extrémité distale de l'aiguille (34) lorsque le stylet (14) est dans la configuration rétractée et dans la configuration déployée, le passage pour fluide (56) étant configuré de telle sorte que le fluide dans le passage pour fluide (56) s'écoule entre la surface extérieure du stylet (46) et la surface intérieure de l'aiguille (28);
dans lequel la pointe distale aiguisée (40) permet à l'aiguille hypodermique (12) de perforer facilement la peau d'un patient afin d'accéder à l'anatomie humaine; et
l'instrument médical (10) est configuré de telle sorte que, contrairement à la chirurgie ouverte, y compris les interventions chirurgicales moins invasives qui utilisent des incisions plus petites de l'ordre de 4 mm ou plus, un point d'entrée utilisé pour effectuer l'intervention avec l'instrument médical (10) se limite uniquement à une piqûre d'aiguille hypodermique présentant une dimension transversale maximale inférieure à 3,5 mm.

2. Instrument médical (10) selon la revendication 1, dans lequel l'aiguille hypodermique (12) est configurée pour se conformer à une taille définie par le calibre Birmingham, et dans lequel l'aiguille hypodermique (12) est plus petite qu'une aiguille de calibre 14 telle que définie par le calibre Birmingham et plus grande qu'une aiguille de calibre 28 telle que définie par le calibre Birmingham.

3. Instrument médical (10) selon la revendication 2, dans lequel l'aiguille hypodermique (12) est plus petite qu'une aiguille de calibre 17 telle que définie par le calibre Birmingham et plus grande qu'une aiguille de calibre 23 telle que définie par le calibre Birmingham.

4. Instrument médical (10) selon la revendication 1, dans lequel l'extrémité proximale du stylet (48) s'étend dans une direction proximale à partir de l'extrémité proximale de l'aiguille (32), et dans lequel l'instrument médical (10) comprend en outre un raccord pour fluide (18) configuré pour être relié de manière amovible à une seringue (86), le raccord pour fluide (18) étant relié à l'extrémité proximale (48) du stylet (14) dans lequel lors de l'utilisation de l'instrument médical (10), le fluide peut être dirigé depuis la seringue (86) à travers l'alésage de l'aiguille (38) et le long du stylet (14) afin que le fluide puisse être évacué par l'extrémité distale de l'aiguille (34) .

5. Instrument médical (10) selon la revendication 4, dans lequel l'instrument médical (10) comprend en outre une collerette (16), la collerette (16) étant reliée de manière fixe à l'extrémité proximale de l'aiguille (32).

6. Instrument médical (10) selon la revendication 5, dans lequel la collerette (16) encercle au moins une partie du stylet (14), le stylet (14) et le raccord pour fluide (18) étant mobiles par rapport à la collerette (16).

7. Instrument médical (10) selon la revendication 6, dans lequel la collerette (16) est configurée pour recevoir au moins une partie du raccord pour fluide (18).

8. Instrument médical (10) selon la revendication 7, dans lequel l'instrument médical (10) comprend en outre un ensemble collerette, l'ensemble collerette comprenant la collerette (16) et au moins un joint torique, le joint torique étant positionné à l'intérieur de la collerette (16).

9. Instrument médical (10) selon la revendication 8, dans lequel la collerette (16) est reliée de manière fluidique au raccord pour fluide (18).

10. Instrument médical (10) selon la revendication 1, dans lequel l'instrument médical (10) est réglable d'une configuration verrouillée à une configuration déverrouillée, le stylet (14) étant fixe par rapport à l'aiguille hypodermique (12) lorsqu'il se trouve dans la configuration verrouillée et mobile par rapport à l'aiguille hypodermique (12) lorsqu'il se trouve dans la configuration déverrouillée.

11. Instrument médical (10) selon la revendication 10, dans lequel l'instrument médical (10) est configuré de telle sorte que le fluide peut s'écouler à travers le passage pour fluide (56) lorsque l'instrument médical (10) se trouve dans la configuration verrouillée et dans la configuration déverrouillée.

12. Instrument médical (10) selon la revendication 11, dans lequel l'instrument médical (10) est configuré de telle sorte que le stylet (14) se trouve dans la configuration rétractée lorsque l'instrument médical (10) est dans la configuration verrouillée.

13. Instrument médical (10) selon la revendication 12, dans lequel le stylet (14) peut tourner autour de l'axe du stylet (52).

14. Instrument médical (10) selon la revendication 13, dans lequel l'instrument médical (10) est configuré pour passer de la configuration verrouillée à la configuration déverrouillée en faisant tourner le stylet (14) autour de l'axe du stylet (52).

15. Instrument médical (10) selon la revendication 1, dans lequel la tête du stylet (44) est configurée pour couper un ligament carpien transversal, et éventuellement: dans lequel le stylet (14) est un composant monolithique solide dépourvu de tout alésage et dans lequel soit: la surface extérieure du stylet (46) du corps du stylet (42) comprend au moins une partie plate s'étendant à travers l'alésage de l'aiguille (38), la partie plate du stylet (14) et la surface intérieure (28) de l'aiguille hypodermique (12) formant ensemble le passage pour fluide (56); ou la surface extérieure du stylet (46) du corps du stylet (42) comprend au moins une zone entaillée s'étendant à travers l'alésage de l'aiguille (38), la zone entaillée du stylet (14) et la surface intérieure (28) de l'aiguille hypodermique (12) formant ensemble le passage pour fluide (56).
